# EUROPEAN PATENT SPECIFICATION

(11) **EP 2 879 196 B1**
(45) Date of publication and mention of the grant of the patent: **19.06.2019**
(21) Application number: 13823334.1
(22) Date of filing: 23.07.2013
(51) Int. Cl.: H01L 51/54, C07D 209/86, C09K 11/06

(54) **LIGHT EMITTING ELEMENT MATERIAL AND LIGHT EMITTING ELEMENT**
MATERIAL FÜR EIN LICHTEMITTIERENDES ELEMENT UND LICHTEMITTIERENDES ELEMENT
MATÉRIAU D'ÉLÉMENT ÉLECTROLUMINESCENT ET ÉLÉMENT ÉLECTROLUMINESCENT

(30) Priority: 25.07.2012 JP 2012164427
(43) Date of publication of application: 03.06.2015
(73) Proprietor: Toray Industries, Inc., Tokyo 103-8666 (JP)
(72) Inventor: NAGAO, Kazumasa, Otsu-shi Shiga 520-8558 (JP); MATSUKI, Shinichi, Otsu-shi Shiga 520-8558 (JP); SAKAINO, Hirotoshi, Otsu-shi Shiga 520-8558 (JP); ARAI, Takeshi, Otsu-shi Shiga 520-8558 (JP); TOMINAGA, Tsuyoshi, Otsu-shi Shiga 520-8558 (JP); KWON, Jinwoo, Seoul 136797 (KR)
(74) Representative: Hoefer & Partner Patentanwälte mbB
(86) International application number: PCT/JP2013/069910
(87) International publication number: WO 2014/017484

(56) References cited:
- WO-A1-2006/025186
- WO-A1-2011/081061
- WO-A1-2012/008281
- WO-A1-2012/090806
- WO-A1-2012/108388
- WO-A1-2012/108881
- WO-A1-2012/121561
- WO-A1-2012/141499
- WO-A1-2012/161382
- WO-A1-2013/032278
- WO-A1-2013/035329
- WO-A1-2013/062075
- WO-A1-2013/085243
- JP-A- H 083 547
- US-A1- 2012 086 329
- US-A1- 2012 175 598

## Description

### TECHNICAL FIELD

The present invention relates to a light-emitting device capable of converting electric energy into light, and a light-emitting device material to be used for the same. More particularly, the present invention relates to a light-emitting device capable of being used for areas such as display devices, flat-panel displays, backlight, lighting, interior design, labels, signboards, electrophotography machines, and light signal generators, and a light-emitting device material to be used for the same.

### BACKGROUND ART

Researches on an organic thin-film light-emitting device in which electrons injected from a cathode and holes injected from an anode emit light when they are recombined in an organic fluorescent body held by both electrodes have been actively conducted in recent years. This light-emitting device is characteristic for high luminance light emission in the form of a thin type and under a low driving voltage, and multicolor light emission due to selection of a fluorescent material, and has been paid attention.

Such researches have undergone many studies for practical use since C. W. Tang et al. of Kodak Co., Ltd. showed that an organic thin-film device emits light at high luminance, and organic thin-film light-emitting devices have steadily come into practical use as they have been employed in main displays of mobile phones, and the like. However, there are still many technical problems and, especially, attainment of both increased efficiency and prolonged life of a device is one of the major problems.

The driving voltage of a device significantly depends on a carrier transporting material that transports carriers such as holes and electrons to an emissive layer. As a material that transports holes (hole transporting material) among the carrier transporting materials, a material having a carbazole skeleton is known (see, for example, Patent Documents 1 to 3). The compound having a carbazole skeleton has a high triplet level, and is therefore known as a host material of an emissive layer (see, for example, Patent Document 4). Patent Documents 5 and 6 also disclose carbazole derivatives for organic electroluminescence devices.

### PRIOR ART DOCUMENT

### PATENT DOCUMENTS

Patent Document 1: Japanese Patent Laid-open Publication
Patent Document 2: Korean Patent Application Publication No. 2010-0079458
Patent Document 3: Korean Patent Application Publication No. 2012-0070507
Patent Document 4: Japanese Patent Laid-open Publication No. 2003-133075.
Patent Document 5: US2012/175598 A1
Patent Document 6: WO2006/025186 A1

### SUMMARY OF THE INVENTION

### PROBLEMS TO BE SOLVED BY THE INVENTION

However, with conventional technologies, it was difficult to reduce the driving voltage of a device sufficiently, and even if the conventional technologies had been capable of reducing the driving voltage, the luminous efficiency and the durable life of a device were insufficient. Thus, technologies capable of realizing all of high luminous efficiency and durable life have not been found yet.

An object of the present invention is to solve such problems with the conventional technologies and provide an organic thin-film light-emitting device that has improved all of luminous efficiency and durable life.

### SOLUTIONS TO THE PROBLEMS

The present invention provides a light-emitting device material including a compound having a carbazole skeleton and represented by the following general formula (1): 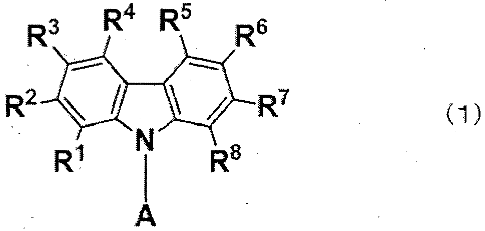 wherein R¹, R² and R⁴ to R⁸ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group, a carbamoyl group, an amino group, a silyl group and -P(=)O R⁹R¹⁰; R⁹ and R¹⁰ are each an aryl group or a heteroaryl group; wherein R³ is a group represented by the following general formula (3), and is directly bonded to the position of R¹⁵; R¹, R² and R⁴ to R⁸ contain none of a dibenzofuran skeleton, a dibenzothiophene skeleton and a carbazole skeleton; and R¹, R² and R⁴ to R¹⁰ contain none of an anthracene skeleton and a pyrene skeleton; A is a group represented by the following general formulae (6) to (9) and (11) to (16) shown below. 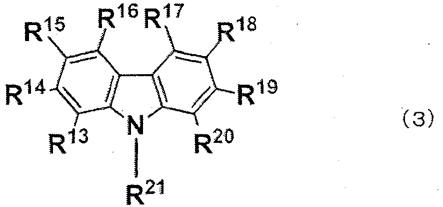 wherein R¹³, R¹⁴ and R¹⁶ to R²⁰ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, halogen, a carbonyl group, a carboxyl group, an oxycarbonyl group, a carbamoyl group, an amino group, a silyl group and -P (=O) R²²R²³; and R²² and R²³ are each an aryl group or a heteroaryl group; wherein R¹⁵ is a group represented by the general formula (1), and is directly bonded to the position of R³; R¹³, R¹⁴ and R¹⁶ to R²⁰ contain none of a dibenzofuran skeleton, a dibenzothiophene skeleton and a carbazole skeleton; and R¹³, R¹⁴ and R¹⁶ to R²⁰, R²² and R²³ contain none of an anthracene skeleton and a pyrene skeleton; wherein in the general formula (1), A and R²¹ are different groups, wherein R²¹ is a substituted or unsubstituted phenyl group or a substituted or unsubstituted naphthyl group and the substituent is an alkyl group or halogen.

### EFFECTS OF THE INVENTION

According to the present invention, there can be provided an organic electric field light-emitting device having high luminous efficiency and sufficient durable life.

The compound represented by the general formula (1) in the present invention is described in detail below. 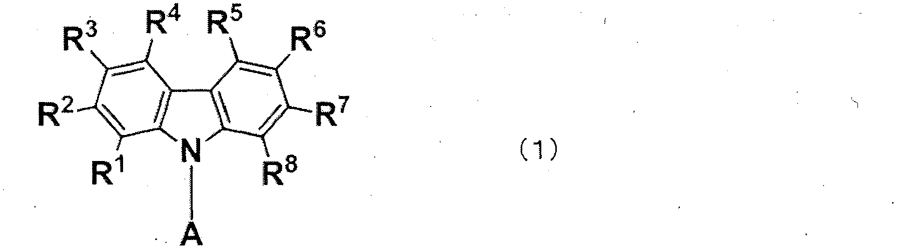 wherein R¹, R² and R⁴ to R⁸ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group, a carbamoyl group, an amino group, a silyl group and -P (=O) R⁹R¹⁰ ; R⁹ and R¹⁰ are each an aryl group or a heteroaryl group, wherein R³ is a group represented by the following general formula (3), and is directly bonded to the position of R¹⁵; R¹, R² and R⁴ to R⁸ contain none of a dibenzofuran skeleton, a dibenzothiophene skeleton and a carbazole skeleton; and R¹, R² and R⁴ to R¹⁰ contain none of an anthracene skeleton and a pyrene skeleton; A is a group represented by the following general formulae (6) to (9) and (11) to (16) shown below. 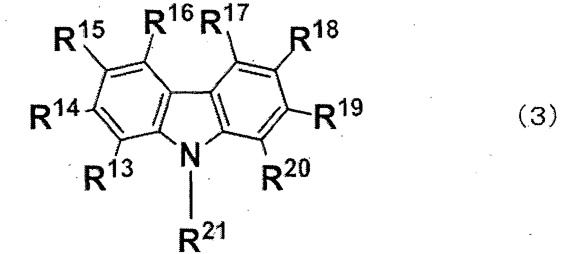 wherein R¹³, R¹⁴ and R¹⁶ to R²⁰ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, halogen, a carbonyl group, a carboxyl group, an oxycarbonyl group, a carbamoyl group, an amino group, a silyl group and -P (=O) R²²R²³; and R²² and R²³ are each an aryl group or a heteroaryl group; wherein R¹⁵ is a group represented by the general formula (1), and is directly bonded to the position of R³; R¹³, R¹⁴ and R¹⁶ to R²⁰ contain none of a dibenzofuran skeleton, a dibenzothiophene skeleton and a carbazole skeleton; and R¹³ , R¹⁴ and R¹⁶ to R²⁰ , R²² and R²³ contain none of an anthracene skeleton and a pyrene skeleton; wherein in the general formula (1), A and R²¹ are different groups, wherein R²¹ is a substituted or unsubstituted phenyl group or a substituted or unsubstituted naphthyl group and the substituent is an alkyl group or halogen.

In all of these substituents, hydrogen may be heavy hydrogen. Hydrogen contained in each of the groups described below may be heavy hydrogen.

The alkyl group denotes a saturated aliphatic hydrocarbon group, such as a methyl group, an ethyl group, a n-propyl group, an isopropyl group, a n-butyl group, a sec-butyl group, or a tert-butyl group, and it may or may not have a substituent. When the alkyl group is substituted, the additional substituent is not particularly limited, examples of the additional substituent may include an alkyl group, an aryl group and a heteroaryl group, and the same holds true in the descriptions below. The number of carbon atoms in the alkyl group is not particularly limited, but from the viewpoints of easy availability and cost, it is normally within the range of 1 or more and 20 or less, more preferably 1 or more and 8 or less.

The cycloalkyl group denotes a saturated alicyclic hydrocarbon group, such as cyclopropyl, cyclohexyl, norbornyl, or adamantyl, and this may or may not have a substituent. The number of carbon atoms in the alkyl group moiety is not particularly limited, but is normally within the range of 3 or more and 20 or less.

The heterocyclic group denotes an aliphatic ring having an atom other than carbon in the ring, such as a pyran ring, a piperidine ring, or a cyclic amide, and this may or may not have a substituent. The number of carbon atoms in the heterocyclic group is not particularly limited, but is normally within the range of 2 or more and 20 or less.

The alkenyl group denotes an unsaturated aliphatic hydrocarbon group containing a double bond, such as a vinyl group, an allyl group, or a butadienyl group, and this may or may not have a substituent. The number of carbon atoms in the alkenyl group moiety is not particularly limited, but is normally within the range of 2 or more and 20 or less.

The cycloalkenyl group denotes an unsaturated alicyclic hydrocarbon group containing a double bond, such as, a cyclopentenyl group, a cyclopentadienyl group, or a cyclohexenyl group, and this may or may not have a substituent. The number of carbon atoms in the cycloalkenyl group is not particularly limited, but is normally within the range of 2 or more and 20 or less.

The alkynyl group denotes an unsaturated aliphatic hydrocarbon group containing a triple bond, such as an ethynyl group, and this may or may not have a substituent. The number of carbon atoms in the alkynyl group moiety is not particularly limited, but is normally within the range of 2 or more and 20 or less.

The alkoxy group denotes a functional group with an aliphatic hydrocarbon group bonded via an ether bond, such as a methoxy group, an ethoxy group, or a propoxy group, and this aliphatic hydrocarbon group may or may not have a substituent. The number of carbon atoms in the alkoxy group is not particularly limited, but is normally within the range of 1 or more and 20 or less.

The alkylthio group is a group in which an oxygen atom of an ether bond in an alkoxy group is substituted with a sulfur atom. The hydrocarbon group of the alkylthio group may or may not have a substituent. The number of carbon atoms in the alkylthio group is not particularly limited, but is normally within the range of 1 or more and 20 or less.

The aryl ether group denotes a functional group with an aromatic hydrocarbon group bonded via an ether bond, such as a phenoxy group, and the aromatic hydrocarbon group may or may not have a substituent. The number of carbon atoms in the aryl ether group is not particularly limited, but is normally within the range of 6 or more and 40 or less.

The aryl thioether group is a group in which an oxygen atom of an ether bond in an aryl ether group is substituted with a sulfur atom. The aromatic hydrocarbon group in the aryl ether group may or may not have a substituent. The number of carbon atoms in the aryl ether group is not particularly limited, but is normally within the range of 6 or more and 40 or less.

The aryl group denotes an aromatic hydrocarbon group, such as a phenyl group, a naphthyl group, a biphenyl group, a fluorenyl group, a phenanthryl group, a triphenylenyl group, or a terphenyl group. The aryl group, may or may not have a substituent. The number of carbon atoms in the aryl group is not particularly limited, but is normally within the range of 6 or more and 40 or less.

The heteroaryl group denotes a cyclic aromatic group having one or a plurality of atoms other than carbon in the ring, such as a furanyl group, a thiophenyl group, a pyridyl group, a quinolinyl group, a pyrazinyl group, a pyriminidinyl group, a triazinyl group, a naphthyridyl group, a benzofuranyl group, a benzothiophenyl group, or an indolyl group, and this may be unsubstituted or substituted. The number of carbon atoms in the heteroaryl group is not particularly limited, but is normally within the range of 2 or more and 30 or less.

The halogen denotes fluorine, chlorine, bromine, or iodine.

The carbonyl group, the carboxyl group, the oxycarbonyl group and the carbamoyl group may or may not have a substituent, and examples of the substituent include an alkyl group, a cycloalkyl group and an aryl group, and these substituents may be further substituted.

The amino group may or may not have a substituent, and examples of the substituent include an aryl group and a heteroaryl group, and these substituents may be further substituted.

The silyl group denotes a functional group having a bond with a silicon atom, such as a trimethylsilyl group, and this may or may not have a substituent. The number of carbon atoms in the silyl group is not particularly limited, but is normally within the range of 3 or more and 20 or less. The number of silicon atoms is normally within the range of 1 or more and 6 or less.

-P(=O)R⁹R¹⁰ and -P(=O)R²²R²³, may or may not have a substituent, and examples of the substituent include an aryl group and a heteroaryl group, and these substituents may be further substituted.

Conventional compounds having a carbazole skeleton do not necessarily have sufficient performance as a light-emitting device material. For example, 4,4'-di(9H-carbazol-9-yl)-1,1'-biphenyl (abbreviated name: CBP) and 1,3-di(9H-carbazol-9-yl)benzene (abbreviated name: mCP) are general-purpose materials as a phosphorescence host material and an exciton blocking material, but both have the problem that the driving voltage is high. In studies on improvement of these materials, the present inventors have focused on the hole transporting ability and electron transporting ability of a compound having a carbazole skeleton. Generally, a compound having a carbazole skeleton has a property to transport charges of both a hole and an electron. In contrast, the present inventors have conceived that conventional compounds have a poor hole transporting ability, and therefore the ratio of holes entering an emissive layer is lower than the ratio of electrons entering from an electron transporting layer, so that the balance of charges in the emissive layer is lost, leading to deterioration of device performance, and based on this hypothesis, the present inventors have invented the compound having a carbazole skeleton and represented by the general formula (1).

The compound represented by the general formula (1) is excellent in hole transporting property as it has a group represented by the general formula (3). The compound having a carbazole skeleton and represented by the general,formula (1) can realize a low driving voltage because R¹⁵ in the general formula (3) is used for coupling to R³ in general formula (1), i.e., a base skeleton, so that the carbazoles coupled to each other exhibit a high hole transporting property, leading to enhancement of hole mobility in a layer. Since carbazole skeletons are coupled, a high triplet level possessed by the carbazole skeleton itself can be maintained, so that easy deactivation can be suppressed, resulting in achievement of high luminous efficiency. Further, the molecule has an asymmetric structure to increase the effect of suppressing an interaction between carbazole skeletons, so that a stable thin film can be formed, leading to enhancement of durability. Therefore, the above-mentioned compound is preferred.

Preferably, the compound having a carbazole skeleton and represented by the general formula (1) contains two carbazole skeletons in the molecule, so that the compound has high thin film stability and excellent heat resistance. When three or more carbazole skeletons are contained, thermal decomposition may occur, and therefore it is preferred that two carbazole skeletons are contained.

Further, R¹, R² and R⁴ to R⁸ contain none of a dibenzofuran skeleton, a dibenzothiophene skeleton and a carbazole skeleton. R¹³, R¹⁴ and R¹⁶ to R²⁰ contain none of a dibenzofuran skeleton, a dibenzothiophene skeleton and a carbazole skeleton. This is because similarly to the above-described reason, when the compound having a carbazole skeleton according to the present invention has as a substituent a carbazole skeleton, or a dibenzofuran skeleton or dibenzothiophene skeleton having a molecular weight comparable to that of the carbazole skeleton, the molecular weight may increase, leading to occurrence of thermal decomposition.

When the compound having a carbazole skeleton and represented by the general formula (1) has as a substituent on N a biphenyl group having at least one substituted or unsubstituted aryl group (A being selected from formulae (11) to (16)), the compound shows an excellent electron blocking property. The presence of the aryl group with which the biphenyl group is further substituted increases the glass transition temperature (Tg) of the compound having a carbazole skeleton and represented by the general formula (1), so that the electron blocking property is remarkably enhanced. As a result, the charge balance in the emissive layer can be improved to enhance light-emitting device performance such as luminous efficiency and life. When the number of the aryl groups is 3 or more, synthesis of the compound becomes difficult because of steric complication. Therefore, the number of the aryl groups is preferably 1 or 2.

The biphenyl group is preferably a meta-biphenyl structure or a para-biphenyl structure from the viewpoint of ease of synthesis.

R¹, R² and R⁴ to R⁸ in the general formula (1) contain none of an anthracene skeleton and a pyrene skeleton. That is, the compound having a carbazole skeleton and represented by the general formula (1) contains none of an anthracene skeleton and a pyrene skeleton in the molecule. This is because the anthracene skeleton and the pyrene skeleton each itself has a low triplet level, and therefore when the compound having a carbazole skeleton according to the present invention has the substituent, the triplet level of the compound is lowered. In the case where the compound having a carbazole skeleton and represented by the general formula (1) is used for the hole transporting layer, luminous efficiency is deteriorated due to occurrence of leakage of triplet excitation energy when the hole transporting layer is in direct contact with an emissive layer containing a triplet emissive dopant if the triplet level is low. In the case where the compound having a carbazole skeleton and represented by the general formula (1) is used for the emissive layer, luminous efficiency is deteriorated because the effect of confining excitation energy by the triplet emissive material cannot be sufficiently exhibited.

In the compound having a carbazole skeleton and represented by the general formula (1), R³ is group represented by the general formula (3). In the group represented by the general formula (3), R¹⁵ is used for coupling to R³. The phrase "R¹⁵ is used for coupling to R³" means that the R³ moiety in the general formula (1) and the R¹⁵ moiety in the general formula (3) are directly bonded to each other.

R³ is a group represented by the general formula (3) because the hole transporting ability is further enhanced.

Further, in the compound having a carbazole skeleton and represented by the general formula (1), A and R²¹ are different groups. In this case, the molecule has an asymmetric structure to increase the effect of suppressing an interaction between carbazole skeletons, so that a more stable thin film can be formed, leading to further enhancement of durability.

The compound represented by the general formula (1) according to the present invention is a compound in which A is a group represented by any one of the general formulae (6) to (9) and (11) to (16). The embodiment wherein A is represented by general formula (10) is not forming part of the present invention.

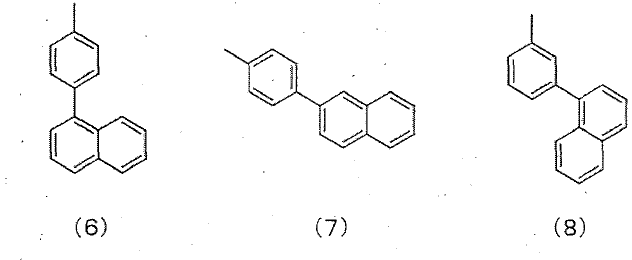

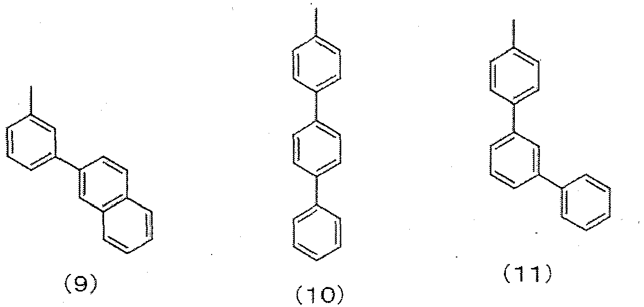

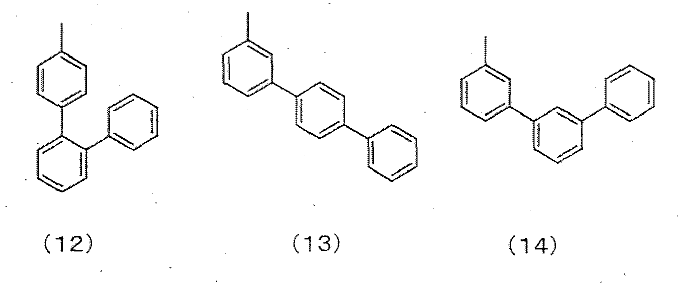

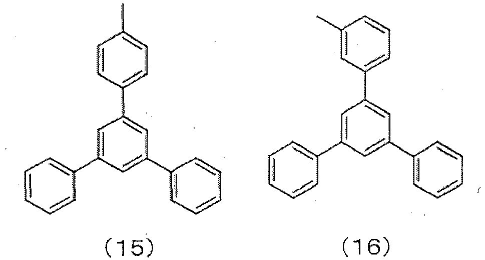

The substituent (other than the group represented by the general formula (3)) possessed by the compound having a carbazole skeleton and represented by the general (formula (1) is preferably hydrogen (including heavy hydrogen), an alkyl group, an aryl group or a heteroaryl group among those described above.

R²¹ is a phenyl or naphthyl group. These groups may be further substituted with an alkyl group or halogen. When a triplet emissive material is used for the emissive layer, the triplet level of the compound having a carbazole skeleton and represented by the general formula (1) according to the present invention becomes a very important value, and therefore R²¹ is preferably a substituted or unsubstituted phenyl group having a high triplet level. In this case, the substituent is preferably one that does not significantly expand the conjugation of the compound or does not lower the triplet level of the compound, and the substituent is an alkyl group or halogen.

The only compounds having a carbazole skeleton and represented by the general formula (1) according to claim 1 of the present inventions are compounds [1], [3], [4], [6], [7], [17], [18], [21], [24], [25], [26], [38] and [39] shown below.

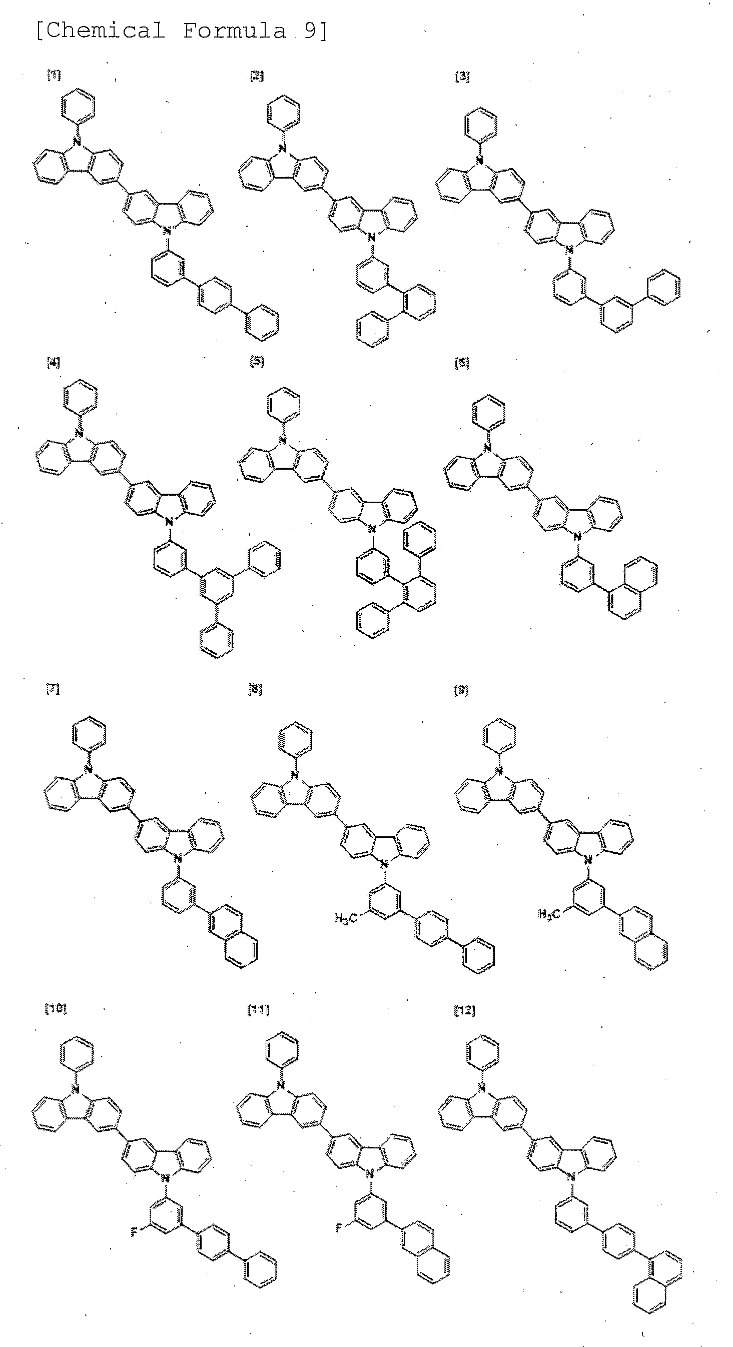

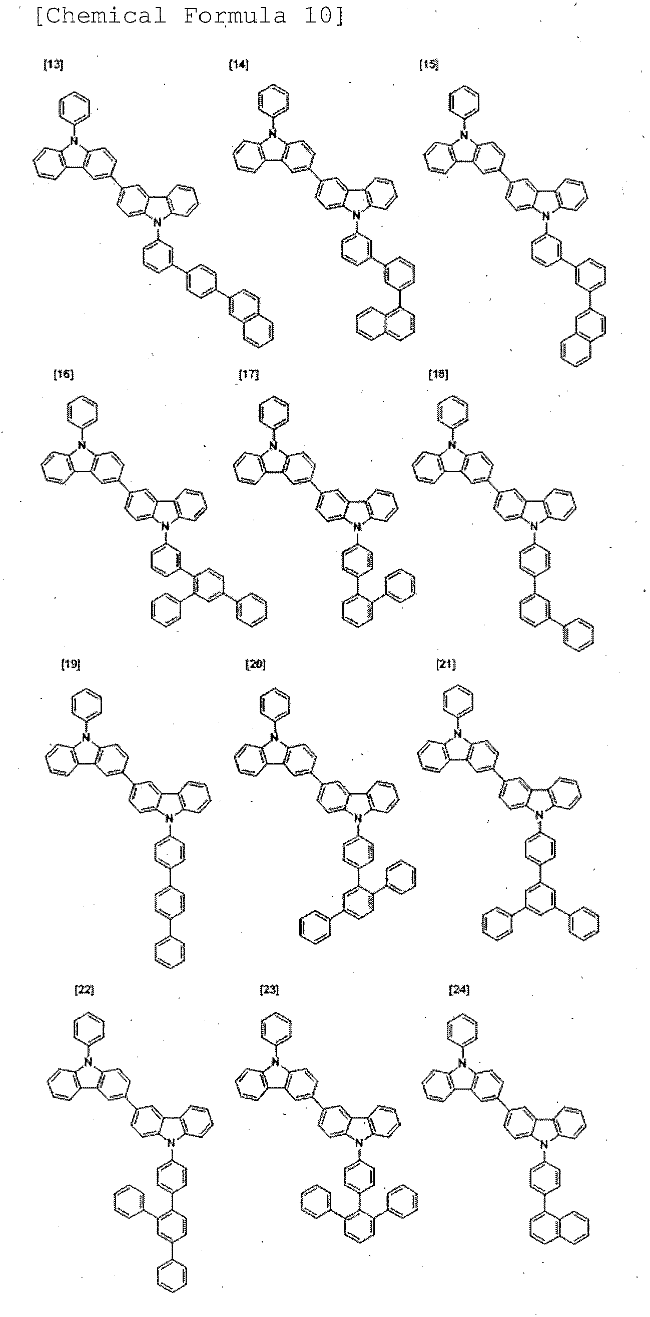

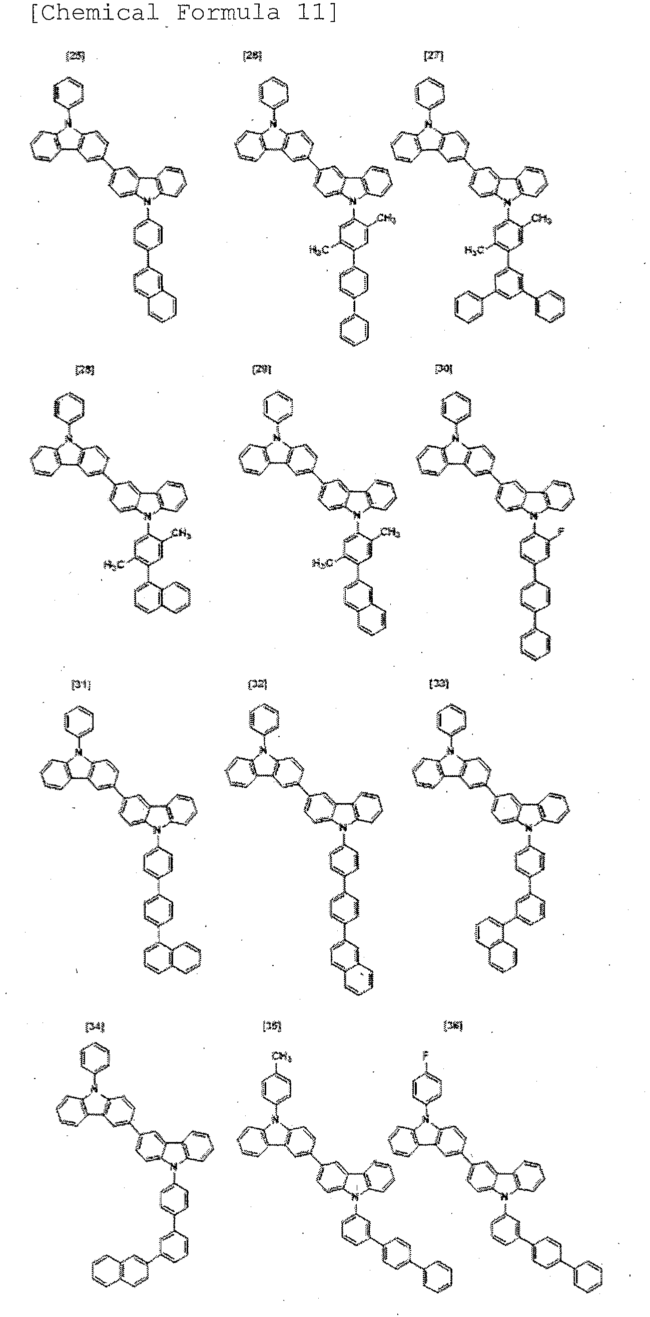

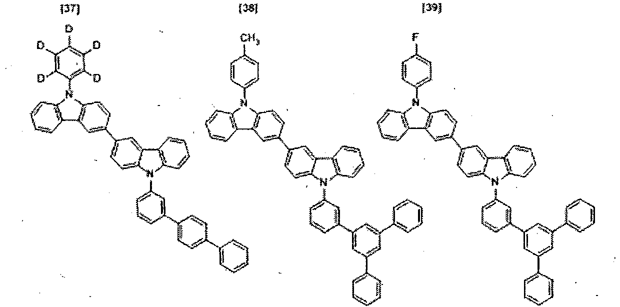

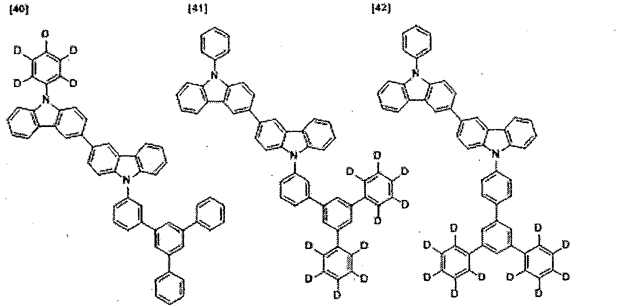

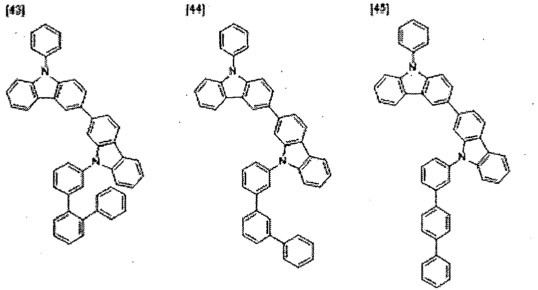

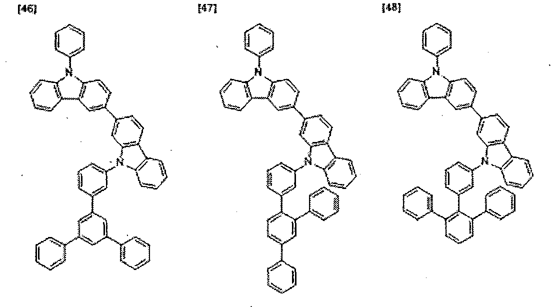

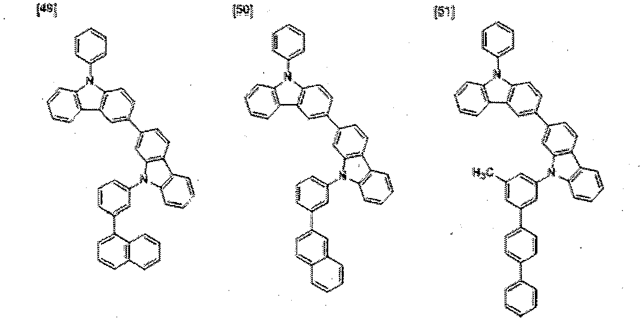

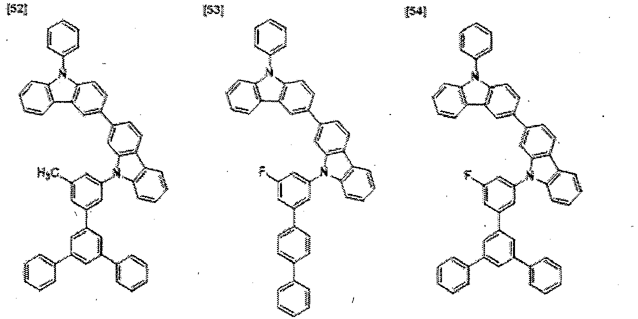

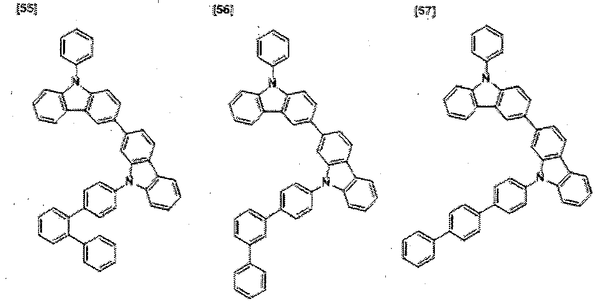

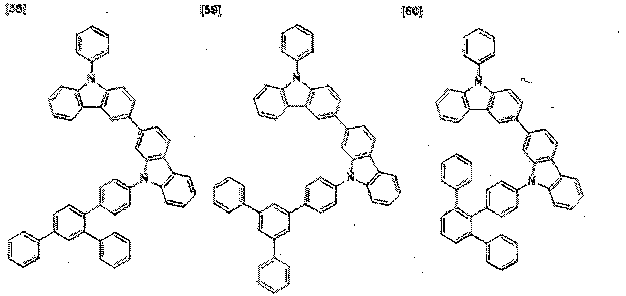

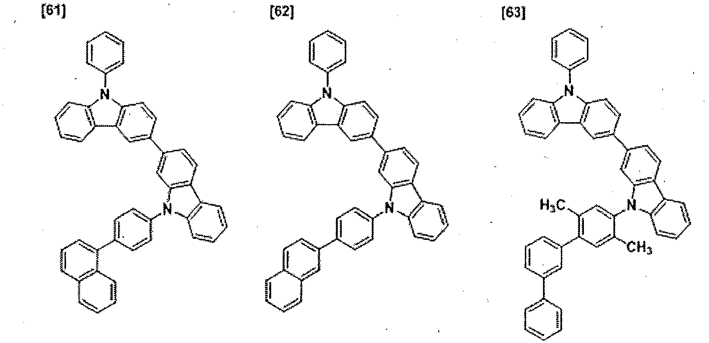

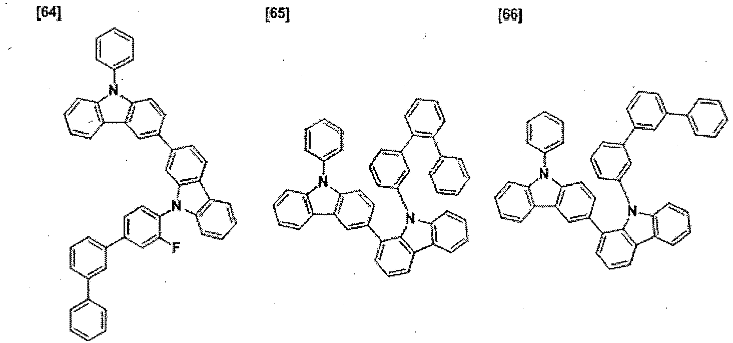

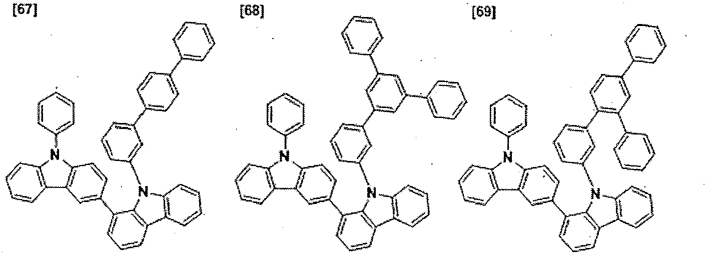

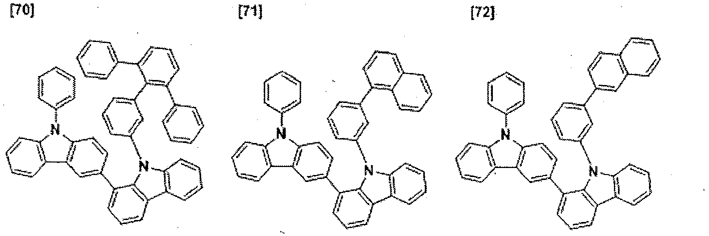

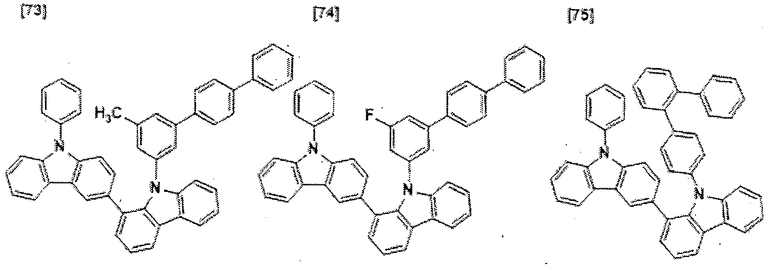

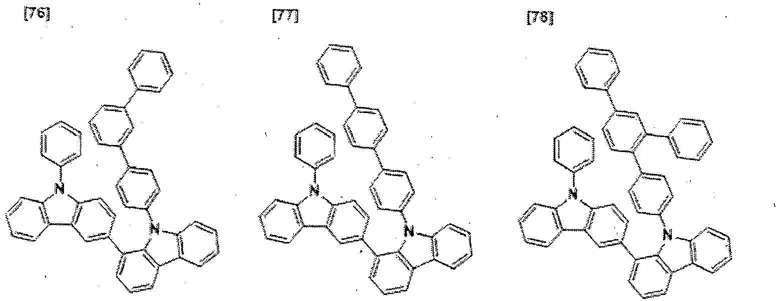

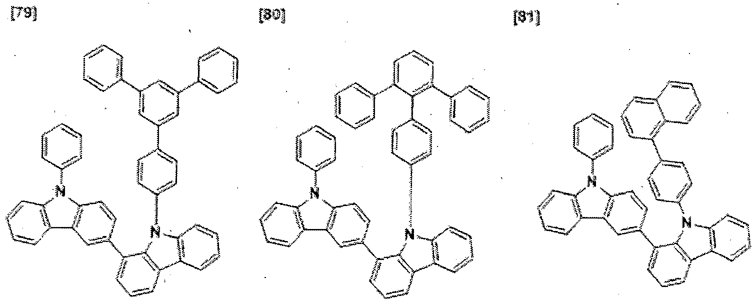

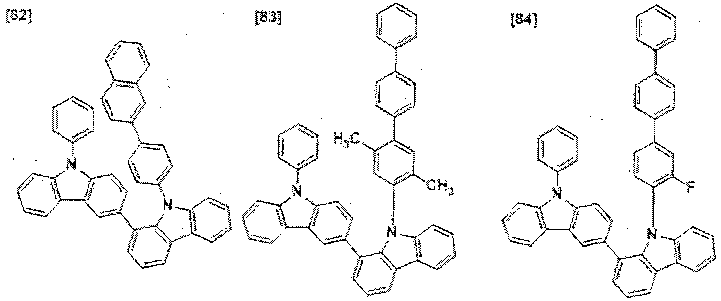

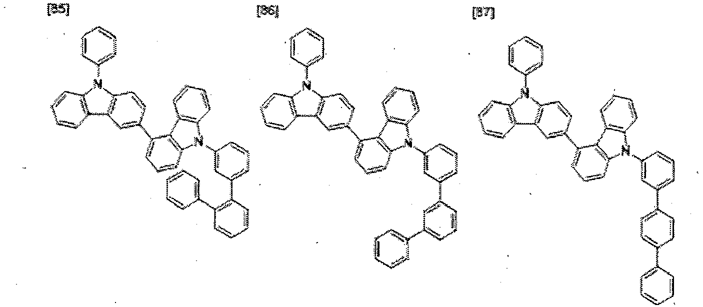

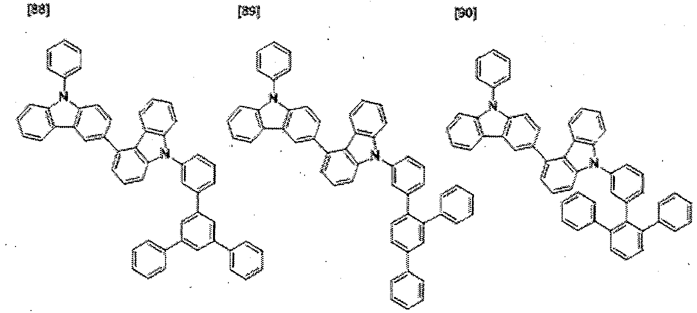

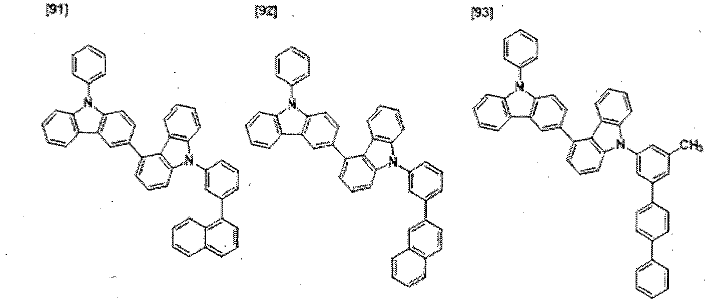

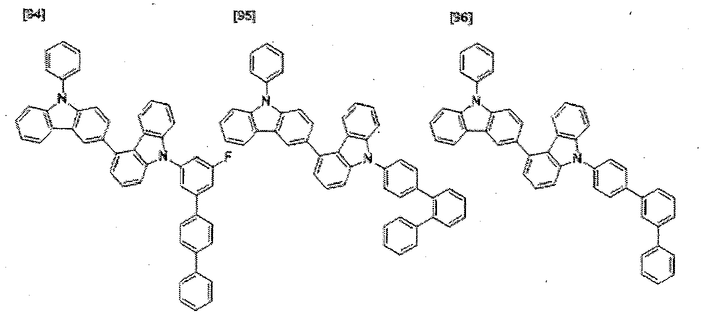

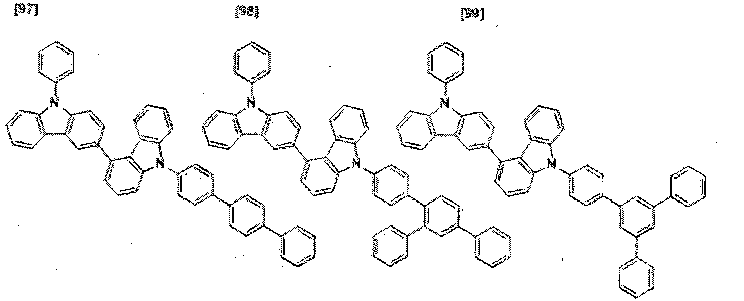

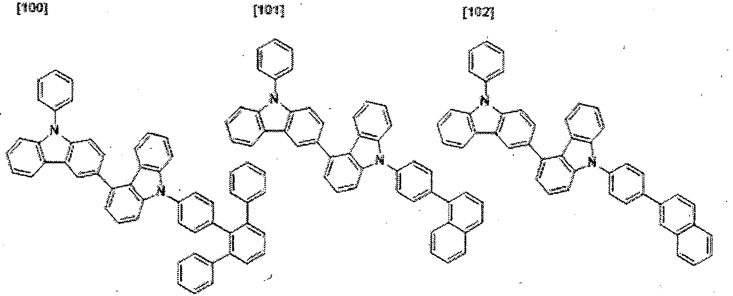

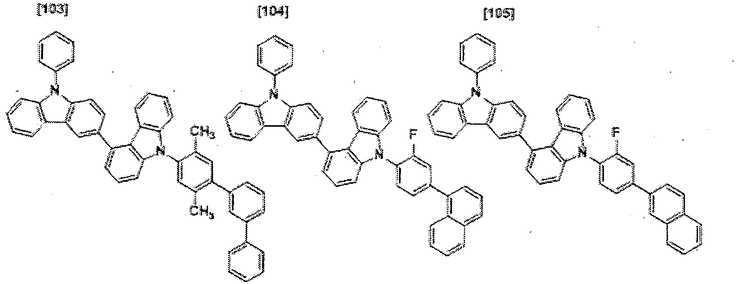

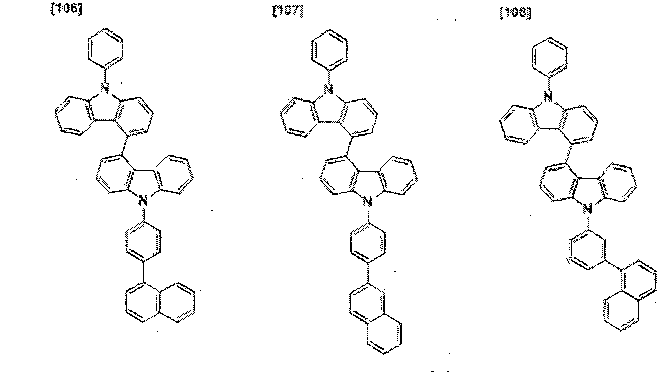

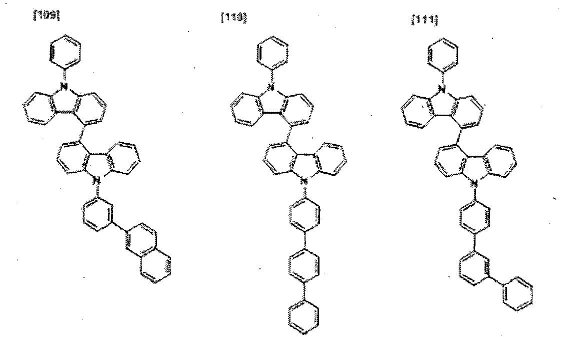

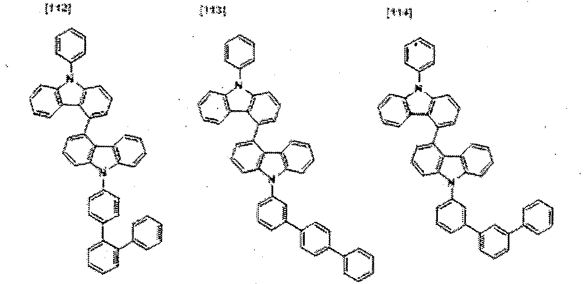

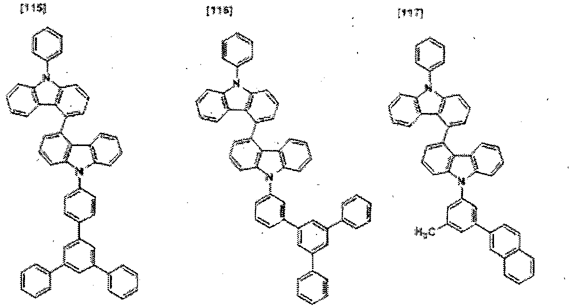

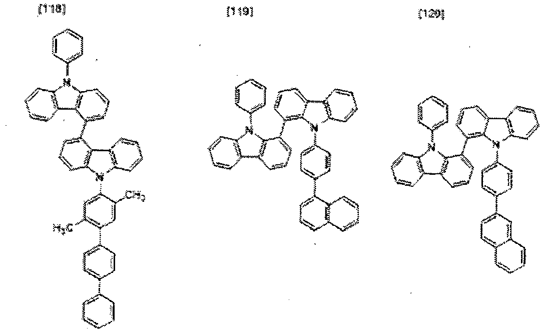

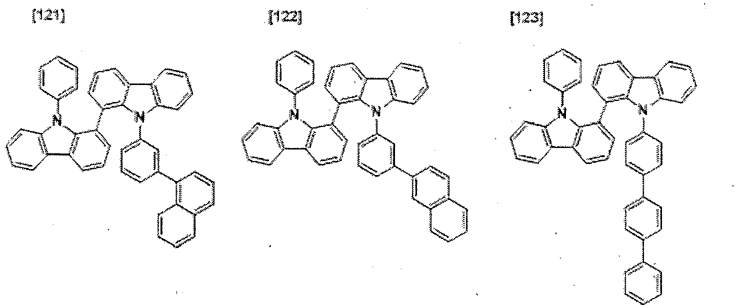

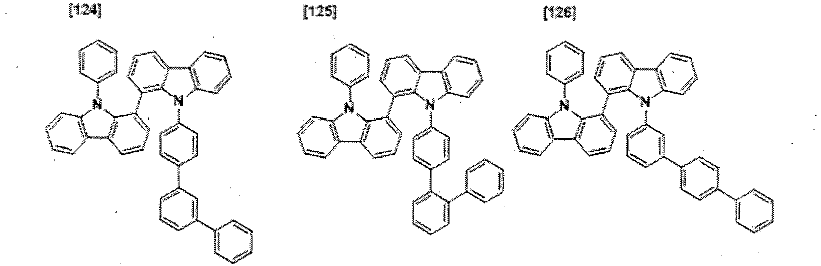

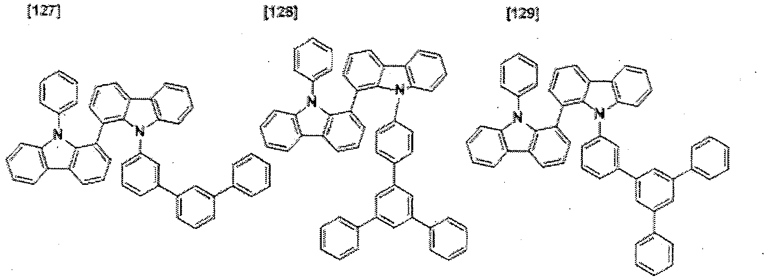

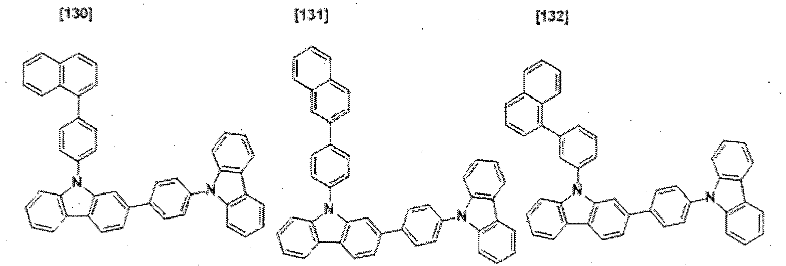

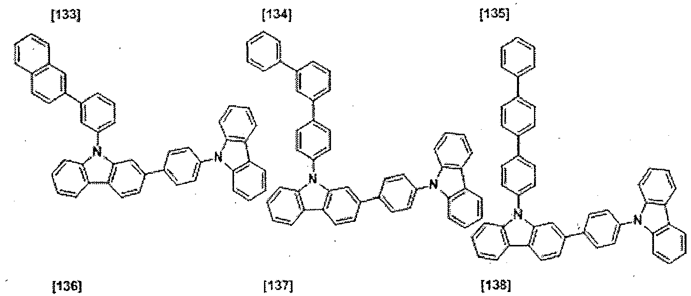

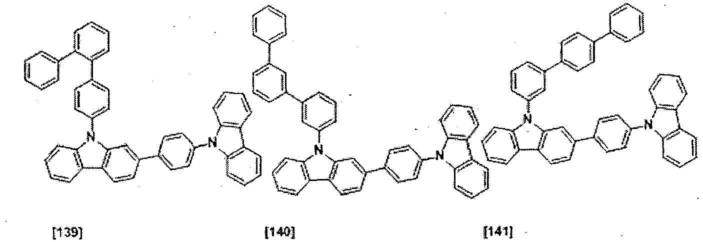

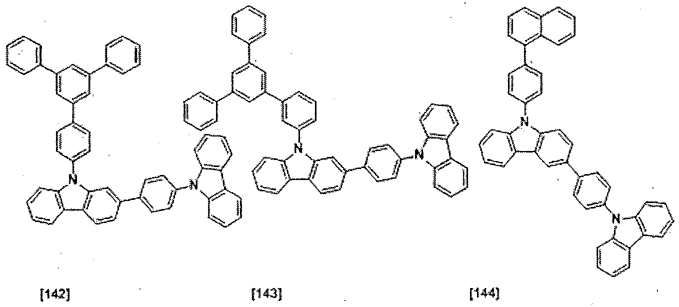

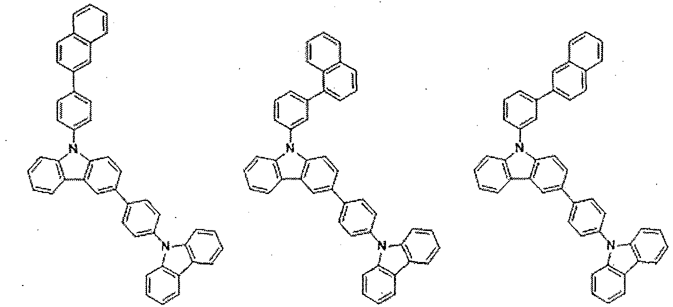

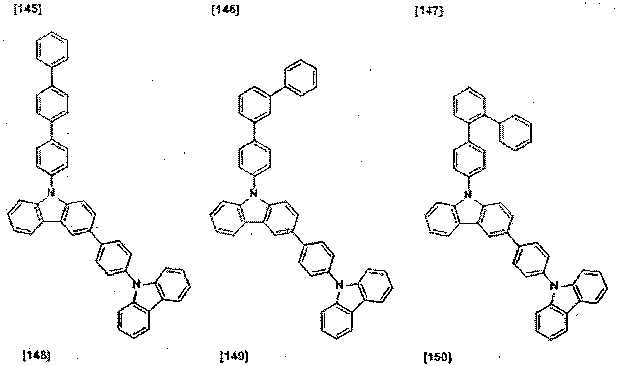

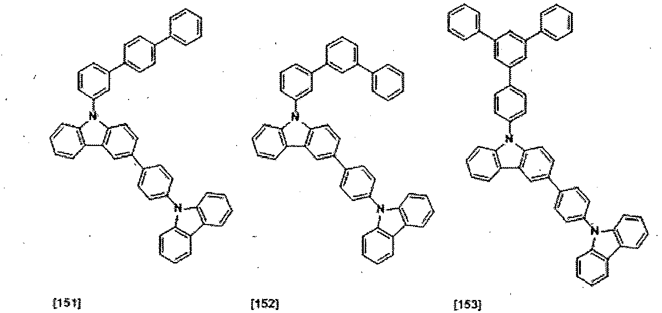

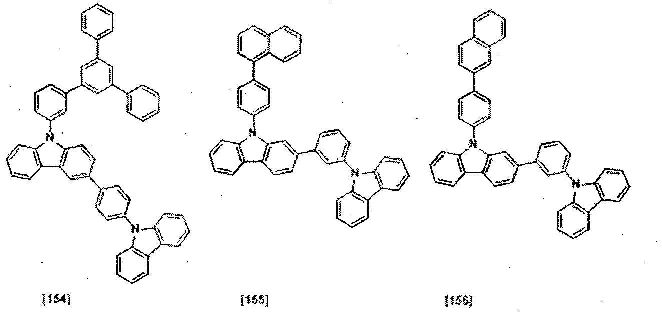

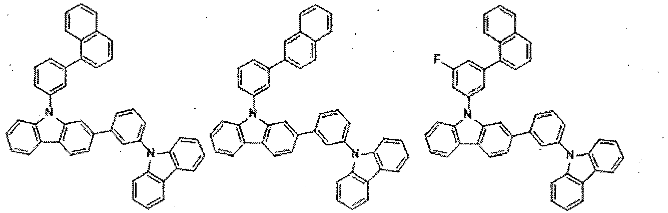

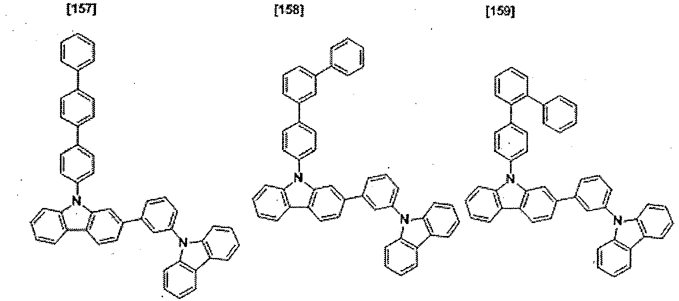

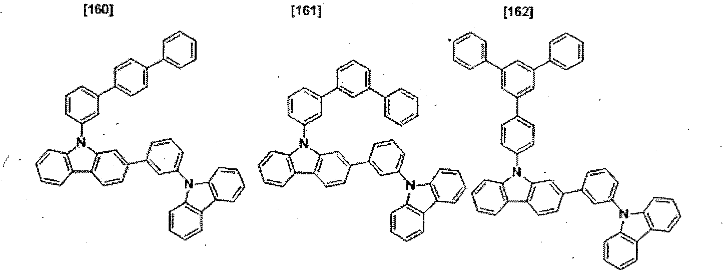

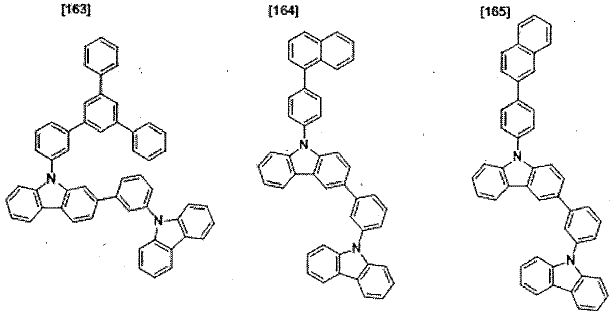

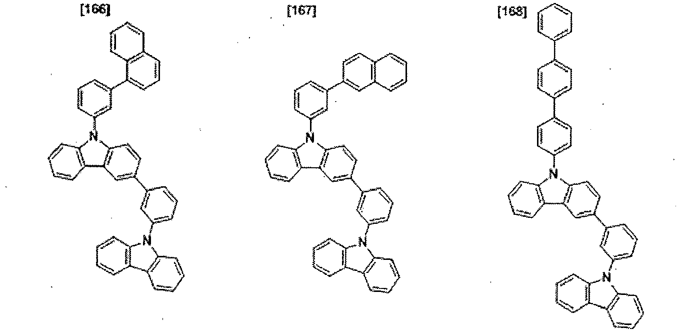

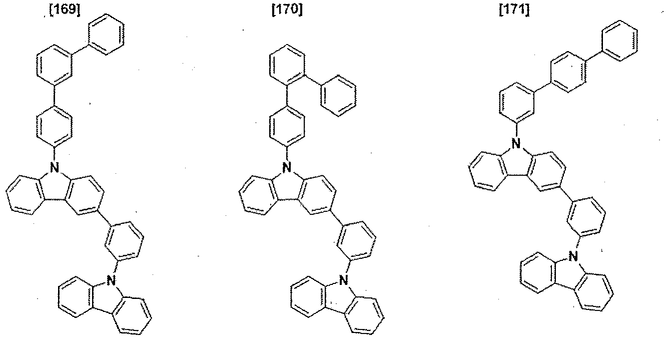

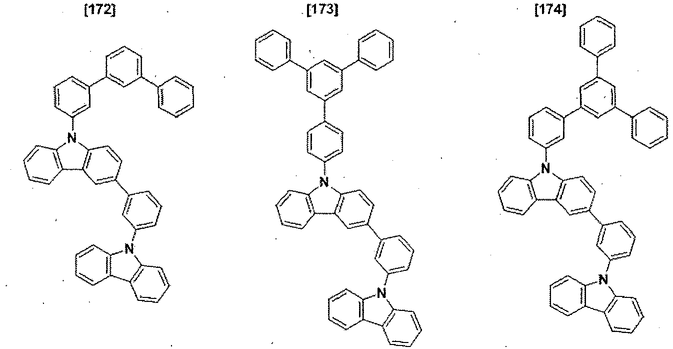

A known method can be used for synthesis of the compound having a carbazole skeleton as described above. Examples of the method for synthesizing a carbazole dimer include, but are not limited to, a method using a coupling reaction of a carbazole derivative with a halide or a triflate using a palladium or copper catalyst. As one example, an example of using 9.-phenylcarbazole-3-boronic acid is shown below.

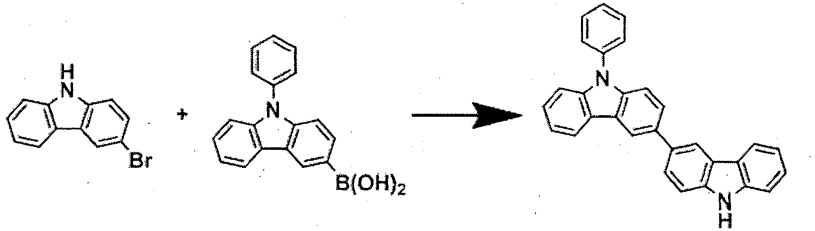

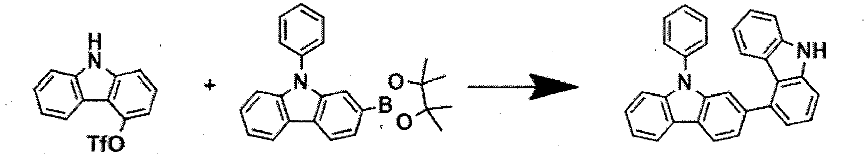

In the reaction described above, leading to a carbazole dimer not forming part of the present invention, the reaction similarly proceeds even when a 9-phenylcarbazole-2-boronic acid ester is used in place of 9-phenylcarbazole-3-boronic acid. In this case, a positional isomer of the carbazole dimer can be synthesized.

Examples of the method for introducing a substituent onto N of carbazole include, but are not limited to, a method using a coupling reaction of a carbazole derivative with a halide using a palladium or copper catalyst.

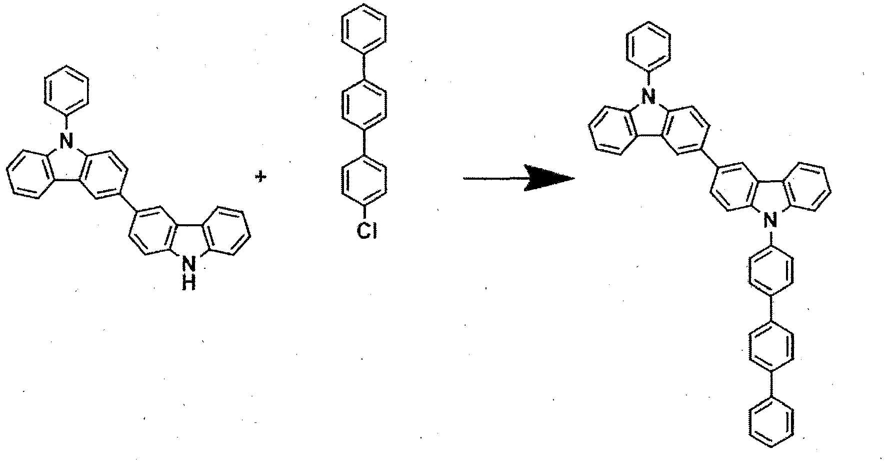

The compound represented by the general formula (1) is used as a light-emitting device material. Here, the light-emitting device material in the present invention denotes a material to be used in any layer of a light-emitting-device and also includes a material to be used in a protective film of a cathode, in addition to materials to be used in a hole injection layer, a hole transporting layer, an emissive layer and/or an electron transporting layer as described later. Use of the compound represented by the general formula (1) in the present invention in any layer of a light-emitting device can afford high luminous efficiency and also can afford a light-emitting device superior in durability.

Next, embodiments of the light-emitting device of the present invention will be described in detail. The light-emitting device of the present invention has an anode and a cathode, and an organic layer interposed between the anode and the cathode, and the organic layer emits light by means of electric energy.

Examples of the layer configuration between the anode and the cathode in such a light-emitting device include, besides a configuration made up of only emissive layer, laminated configurations such as 1) emissive layer/electron transporting layer, 2) hole transporting layer/emissive layer, 3) hole transporting layer/emissive layer/electron transporting layer, 4) hole injection layer/hole transporting layer/emissive layer/electron transporting layer, 5) hole transporting layer/emissive layer/electron transporting layer/electron injection layer, and 6) hole injection layer/hole transporting layer/emissive layer/electron transporting layer/electron injection layer. Each of the layers may be in the form of a single layer or a plurality of layers, and may be doped.

While the compound represented by the general formula (1) may be used for any layer of the layers described above in a light-emitting device, it is particularly suitably used for the hole transporting layer or the emissive layer.

In the light-emitting device of the present invention, the anode and the cathode have a role for supplying a sufficient current for light emission of the device, and it is desirable that at least one of them be transparent or translucent in order to take out light. Usually, the anode formed on a substrate is made to be a transparent electrode.

While the material to be used for an anode is not particularly limited and may be electroconductive metal oxides, such as zinc oxide, tin oxide, indium oxide, tin oxide indium (ITO), and zinc-oxide indium (IZO) ; metals, such as gold, silver, and chromium; inorganic electroconductive substances, such as copper iodide and copper sulfide; or electroconductive polymers, such as polythiophene, polypyrrole, and polyaniline as long as being a material that is capable of injecting holes into an organic layer efficiently and that is transparent or translucent in order to take out light, use of ITO glass or NESA glass is particularly desirable. These electrode materials may be used alone, or a plurality of materials may be used in lamination or in admixture. Since it is favorable that a sufficient current for light emission of the device can be supplied, the resistance of a transparent electrode is not limited, but from the viewpoint of the power consumption of the device, a low resistance is desirable. For example, an ITO substrate having a resistance of 300 Ω/□ or lower functions as a device electrode, but since currently, it is possible to supply a substrate having a resistance of about 10 Ω/□, it is particularly desirable to use a substrate having a low resistance of 20 Ω/□ or lower. The thickness of ITO can be arbitrarily selected according to a resistance value, but ITO is usually used at a thickness of between 50 to 300 nm in many cases.

In addition, in order to retain the mechanical strength of the light-emitting device, it is preferred to form the light-emitting device on a substrate. As the substrate, a glass substrate such as soda glass or alkali-free glass is suitably used. Since it is favorable that the thickness of a glass substrate has a sufficient thickness for retaining the mechanical strength, a thickness of 0.5 mm or more is sufficient. Regarding the material of glass, since it is preferred that the amount of ions eluted from glass is small, alkali-free glass is more preferable. Alternatively, since soda lime glass provided with a barrier coating such as SiO₂ is commercially available, it can also be used. Further, as far as a first electrode stably functions, it is not necessary that the substrate is glass and, for example, the anode may be formed on a plastic substrate. Examples of a method of forming an ITO film include, but are not particularly limited to, an electron beam method, a sputtering method, and a chemical reaction method.

A material to be used in the cathode is not particularly limited, as far as it is a substance which can efficiently inject electrons into the emissive layer. Generally, metals such as platinum, gold, silver, copper, iron, tin, aluminum, and indium, or alloys or multilayer lamination of these metals with metals having a low work function such as lithium, sodium, potassium, calcium and magnesium are preferred. Among them, as a main component, aluminum, silver, and magnesium are preferred from the viewpoints of electric resistance value, easiness of making a film, stability of a film, and luminous efficiency. In particular, it is preferred that the material is constituted by magnesium and silver because electron injection into the electron transporting layer and the electron injection layer in the present invention becomes easy, and low voltage driving becomes possible.

Further, preferable examples include lamination of metals such as platinum, gold, silver, copper, iron, tin, aluminum, and indium, or alloys using these metals, inorganic substances such as silica, titania, and silicon nitride, and organic polymer compounds such as polyvinyl alcohol, polyvinyl chloride, and a hydrocarbon-based polymer compound as a protective film layer on the cathode for protecting the cathode. However, in the case of a device structure for taking out light from the cathode side (top emission structure), the protective film layer is selected from materials having light permeability in a visible light region. Examples of a method for preparation of these electrodes include, but are not particularly limited to, resistance heating, electron beam, sputtering, ion plating and coating.

The hole injection layer is a layer that is inserted between an anode and a hole transporting layer. The hole injection layer may be in the form of a single layer, or a plurality of layers laminated. It is preferred that the hole injection layer is present between a hole transporting layer and an anode because this successfully results in lower voltage driving, increased durable life, and enhancement in luminous efficiency due to enhancement in the carrier balance of a device.

The material to be used for the hole injection layer is not particularly limited, and, for example, benzidine derivatives such as
4,4'-bis(N-(3-methylphenyl)-N-phenylamino)biphenyl (TPD), 4,4'-bis(N-(1-naphthyl)-N-phenylamino)biphenyl (NPD), 4,4'-bis(N,N-bis(4-biphenylyl)amino)biphenyl (TBDB), bis(N,N'-diphenyl-4-aminophenyl)-N,N-diphenyl-4,4'-diamino-1,1'-biphenyl (TPD232) and
N⁴,N^{4'}-([1,1'-biphenyl]-4,4'-diyl)bis(N⁴, N^{4'},N^{4'}-triphenyl-[1 ,1'-biphenyl]-4,4'-diamine); materials called starburst arylamines, such as 4,4',4"-tris(3-methylphenyl(phenyl) amino)triphenylamine (m-MTDATA) and
4,4',4"-tris(1-naphthyl(phenyl)amino)triphenylamine (1-TNATA); biscarbazole derivatives such as bis(N-arylcarbazole) or bis(N-alkylcarbazole); monocarbazole derivatives such as
N-([1,1'-biphenyl]-4-yl)-9,9-dimethyl-N-(4-(9-phenyl-9H-car bazol-3-yl)phenyl)-9H-fluorene-2-amine; heterocyclic compounds such as pyrazoline derivatives, stilbene-based compounds, hydrazone-based compounds, benzofuran derivatives, thiophene derivatives, oxadiazole derivatives, phthalocyanine derivatives and porphyrin derivatives; and such polymers as polycarbonates and styrene derivatives having the aforementioned monomers on their side chains, polythiophene, polyaniline, polyfluorene, polyvinylcarbazole and polysilane are used. The compound represented by the general formula (1) can also be used. Among them, benzidine derivatives, starburst arylamine materials and monocarbazole derivatives are more preferably used from the viewpoint of having a lower HOMO level than the compound represented by the general formula (1) and injecting and transporting holes smoothly from an anode to a hole transporting layer.

These materials may be used alone, or may be used in admixture of two or more thereof. A plurality of materials may be laminated to form a hole injection layer. Further, it is more preferred that the hole injection layer is formed of an acceptor compound alone, or the hole injection material described above is used with the material doped with an acceptor compound used with the above-mentioned hole injection material doped with an acceptor compound because the above-described effects are more remarkably obtained. The acceptor compound is a material that forms a charge transfer complex with an adjacent hole transporting layer when the compound is used as a single-layer film, or forms a charge transfer complex with a material that constitutes a hole injection layer when the compound is used while being doped into the material. Use of such a material enhances the conductivity of a hole injection layer and contributes more to lower the driving voltage of a device, thereby affording effects such as enhancement in luminous efficiency and enhancement in durable life.

Examples of the acceptor compound include metal chlorides such as iron (III) chloride, aluminum chloride, gallium chloride, indium chloride, and antimony chloride, metal oxides such as molybdenum oxide, vanadium oxide, tungsten oxide, and ruthenium oxide, and charge transfer complexes such as tris(4-bromophenyl)aminium hexachloroantimonate (TBPAH). Moreover, organic compounds having a nitro group, a cyano group, halogen, or a trifluoromethyl group in the molecule, quinone-based compounds, acid anhydride-based compounds, and fullerene can also be used suitably. Specific examples of such compounds include hexacyanobutadiene, hexacyanobenzene, tetracyanoethylene, tetracyanoquinodimethane (TCNQ), tetrafluorotetracyanoquinodimethane (F4-TCNQ), radialene derivatives such as 4,4',4"-((1E,1'E,1"E)-cyclopropane -1,2,3-triylidenetris(cyanomethanylylidene))tris(2,3,5,6-te trafluorobenzonitrile), p-fluoranil, p-chloranil, p-bromanil, p-benzoquinone, 2,6-dichlorobenzoquinone, 2,5-dichlorobenzoquinone, tetramethylbenzoquinone, 1,2,4,5-tetracyanobenzene,o-dicyanobenzene, p-dicyanobenzene, dipyrazino[2,3-f:2',3'-h]quinoxaline -2,3,6,7,10,11-hexacarbonitrzle(HAT(CN)₆), 1,4-dicyanotetrafluorobenzene, 2,3-dichloro-5,6-dicyanobenzoquinone, p-dinitrobenzene, m-dinitrobenzene, o-dinitrobenzene, p-cyanonitrobenzene, m-cyanonitrobenzene, o-cyanonitrobenzene, 1,4-naphthoquinone, 2,3-dichloronaphthoquinone, 1-nitronaphthalene, 2-nitronaphthalene, 1,3-dinitronaphthalene, 1,5-dinitronaphthalene, 9-cyanoanthracene, 9-nitroanthracene, 9,10-anthraquinone, 1,3,6,8-tetranitrocarbazole, 2,4,7-trinitro-9-fluorenone, 2,3,5,6-tetracyanopyridine, maleic anhydride, phthalic anhydride, C60, and C70.

Of these, metal oxides and cyano group-containing compounds are preferred because they can be easily handled and deposited, and therefore the above-described effects can be obtained easily. In either of the case where a hole injection layer is formed of an acceptor compound alone or the case where a hole injection layer is doped with an acceptor compound, the hole injection layer may be a single layer or may be formed of a plurality of layers laminated.

The hole transporting layer is a layer that transports holes injected from an anode to an emissive layer. The hole transporting layer may be in the form of a single layer, or a plurality of layers laminated.

The compound represented by the general formula (1) has an ionization potential of 5.3 to 6.0 eV (measured value of deposited film using AC-2 (RIKEN KEIKI Co., Ltd.)), a high triplet level, a high hole transporting property and thin film stability, and is therefore preferably used for the hole injection layer and hole transporting layer of the light-emitting device. The compound represented by the general formula (1) has a larger energy gap as compared to a conventional hole transporting material having a benzidine skeleton, and therefore has a high LUMO level and is excellent in electron blocking property. Further, the compound represented by the general formula (1) is preferably used as a hole transporting material of a device using a triplet emissive material. This is because a conventional hole transporting material having a benzidine skeleton has a low triplet level, so that when the material is in direct contact with an emissive layer containing a triplet emissive dopant, leakage of triplet excitation energy occurs and luminous efficiency is deteriorated, but the compound represented by the general formula (1) has a high triplet level, so that the above-mentioned problem does not occur.

When the device includes a plurality of hole transporting layers, it is preferred that a hole transporting layer containing the compound represented by the general formula (1) is in direct contact with the emissive layer. This is because the compound represented by the general formula (1) has a high electron blocking property, and therefore can prevent the invasion of electrons flowing out of the emissive layer. Further, the compound represented by the general formula (1) has a high triplet level, and therefore also has an effect of confining the excitation energy of a triplet emissive material. Accordingly, also when the emissive layer contains a triplet emissive material, it is preferred that a hole transporting layer containing the compound represented by the general formula (1) is in direct contact with the emissive layer. Particularly, when an electron transporting host material is used for the emissive layer, excitons generated in the emissive layer are likely leaked to the hole transporting layer side, and therefore the compound of the present invention having a high triplet level is preferably used.

The hole transporting layer may be formed of only the compound represented by the general formula (1), or other materials may be mixed therein as long as the effects of the present invention are not impaired. In this case, examples of the other materials to be used include benzidine derivatives such as 4,4'-bis(N-(3-methylphenyl)-N-phenylamino)biphenyl (TPD), 4,4'-bis(N-(1-naphthyl)-N-phenylamino)biphenyl (NPD), 4,4'-bis(N,N-bis(4-biphenylyl)amino)biphenyl (TBDB), bis(N,N'-diphenyl-4-aminophenyl)-N,N-diphenyl-4,4'-diamino-1,1'-biphenyl (TPD232) and N⁴,N^{4'}-([1,1'-biphenyl]-4,4'-diyl) bis (N⁴,N^{4'},N^{4'}-triphenyl-[1,1'-biphenyl]-4,4'-diamine); materials called starburst arylamines, such as 4,4',4"-tris (3-methylphenyl(phenyl)amino)triphenylamine (m-MTDATA) and 4,4',4"-tris(1-naphthyl(phenyl)amino)triphenylamine (1-TNATA); biscarbazole derivatives such as bis(N-arylcarbazole) or bis(N-alkylcarbazole); monocarbazole derivatives such as N-([1,1'-biphenyl]-4-yl)-9,9-dimethyl -N-(4-(9-phenyl-9H-carbazol-3-yl)phenyl)-9H-fluorene-2-amin e; heterocyclic compounds such as pyrazoline derivatives, stilbene-based compounds, hydrazone-based compounds, benzofuran derivatives, thiophene derivatives, oxadiazole derivatives, phthalocyanine derivatives and porphyrin derivatives; and such polymers as polycarbonates and styrene derivatives having the aforementioned monomers on their side chains, polythiophene, polyaniline, polyfluorene, polyvinylcarbazole and polysilane.

The emissive layers may be in the form of a single layer or a plurality of layers, each of which is formed of an emissive material (host material, dopant material), and this may be a mixture of the host material and the dopant material, or the host material alone. That is, in the light-emitting device of the present invention, only the host material or the dopant material may emit light, or both of the host material and the dopant material emit light, in each emissive layer. From the viewpoints that electric energy is efficiently utilized and light emission at high color purity is obtained, it is preferred that the emissive layer includes a mixture of the host material and the dopant material. In addition, the host material and the dopant material may be one kind or a combination of a plurality of kinds, respectively. The dopant material may be contained in a whole host material, or may be partially contained therein. The dopant material may be laminated, or may be dispersed. The dopant material can control an emitted color. When the amount of the dopant material is too large, concentration quenching occurs, and therefore the dopant material is preferably used in an amount of 30% by weight or less, further preferably 20% by weight or less based on the host material. As a doping method, the dopant material can be co-deposited with the host material, or the dopant material, may be mixed with the host material in advance to be deposited simultaneously.

In addition to the compound represented by the general formula (1), examples of the emissive material that can be used include, but are not particularly limited to, fused ring derivatives such as anthracene and pyrene, metal chelated oxinoid compounds including tris(8-quinolinolato)aluminum, bisstyryl derivatives such as bisstyrylanthracene derivatives and distyrylbenzene derivatives, tetraphenylbutadiene derivatives, indene derivatives, coumarin derivatives, oxadiazole derivatives, pyrrolopyridine derivatives, perinone derivatives, cyclopentadiene derivatives, oxadiazole derivatives, thiadiazolopyridine derivatives, dibenzofuran derivatives, dibenzothiophene derivatives, carbazole derivatives, and indolocarbazole derivatives and, as a polymer series, polyphenylenevinylene derivatives, polyparaphenylene derivatives, and polythiophene derivatives, which have hitherto been known as a light emitting body.

The host material contained in the emissive material is not needed to be limited to only one kind of compound, and a plurality of compounds of the present invention may be used in admixture, or one or more of other host materials may be used in admixture. Further, these materials may be laminated. The host material is not particularly limited, and examples of the host material which can be used include, but are not particularly limited to, compounds having a fused aryl ring such as naphthalene, anthracene, phenanthrene, pyrene, chrysene, naphthacene, triphenylene, perylene, fluoranthene, fluorene and indene, and derivatives thereof, aromatic amine derivatives such as N,N'-dinaphthyl-N,N'-diphenyl-4,4'-diphenyl -1,1'-diamine, metal chelated oxinoid compounds including tris (8-quinolinato) aluminum (III), bisstyryl derivatives such as distyrylbenzene derivatives, tetraphenylbutadiene derivatives, indene derivatives, coumarin derivatives, oxadiazole derivatives, pyrrolopyridine derivatives, perinone derivatives, cyclopentadiene derivatives, pyrrolopyrrole derivatives, thiadiazolopyridine derivatives, dibenzofuran derivatives, dibenzothiophene derivatives, carbazole derivatives, indolocarbazole derivatives, pyrimidine derivatives and triazine derivatives and, as a polymer series, polyphenylenevinylene derivatives, polyparaphenylene derivatives, polyfluorene derivatives, polyvinylcarbazole derivatives, and polythiophene derivatives. Particularly, as a host to be used when the emissive layer performs triplet emission (phosphorescence emission), metal chelated oxinoid compounds, dibenzofuran derivatives, dibenzothiophene derivatives, carbazole derivatives, indolocarbazole derivatives, pyrimidine derivatives, triazine derivatives, triphenylene derivatives or the like is suitably used.

Particularly, the compound represented by the general formula (1) has an ionization potential of 5.3 to 6.0 eV (measured value of deposited film using AC-2 (RIKEN KEIKI Co. , Ltd.)), a high triplet level, a high hole transporting property and thin film stability, and is therefore preferably used for the emissive layer of the light-emitting device. The compound represented by the general formula (1) has a larger energy gap as compared to a conventional material having a benzidine skeleton and having a high hole transporting property, and therefore has a high LUMO level and is excellent in electron blocking property. Further, the compound represented by the general formula (1) is preferably used as a host material of a device using a triplet emissive material. This is because the conventional material has a low triplet level, so that when the material is in direct contact with an emissive layer containing a triplet emissive dopant, leakage of triplet excitation energy occurs and luminous efficiency is deteriorated, but the compound represented by the general formula (1) has a high triplet, level, so that the above-mentioned problem does not occur. The compound represented by the general formula (1) shows proper hole injection/transporting properties as described above, and also has an improved electron blocking property, and therefore can be used as a hole transporting host. Further, use of the compound in combination with an electron transporting host is preferred because the number of carriers in the emissive layer increases, so that the probability of recombination is increased, resulting in enhancement of luminous efficiency. The electron transporting host material is not particularly limited, but a carbazole compound containing a pyrimidine skeleton or a triazine skeleton, or a compound having a carbazole site is preferably used.

The dopant material contained in the emissive material is not particularly limited, and examples thereof include compounds having an aryl ring, such as naphthalene, anthracene, phenanthrene, chrysene, fluorene, benzofluorene, pyrene, triphenylene, perylene, fluorene and indene, and derivatives thereof (e.g., 2-(benzothiazol-2-yl)-9,10-diphenylanthracene and 5,6,11,12-tetraphenylnaphthacene); compounds having a heteroaryl ring, such as furan, pyrrole, thiophene, silole, 9-silafluorene, 9,9'-spirobisilafluorene, benzothiophene, benzofuran, indole, dibenzothiophene, dibenzofuran, imidazopyridine, phenanthroline, pyrazine, naphthyridine, quinoxaline, pyrrolopyridine and thioxanthene, and derivatives thereof; distyrylbenzene derivatives; aminostyryl derivatives such as 4,4'-bis(2-(4-diphenylaminophenyl) ethenyl)biphenyl and 4,4'-bis(N-(stilben-4-yl) -N-phenylamino)stilbene; aromatic acetylene derivatives; tetraphenylbutadiene derivatives; stilbene derivatives; aldazine derivatives; pyrromethene derivatives; diketopyrrolo[3,4-c]pyrrole derivatives; coumarin derivatives such as 2,3,5,6-1H,4H-tetrahydro -9-(2'-benzothiazolyl)quinolizino[9,9a,1-gh]coumarin; azole derivatives such as imidazole, thiazole, thiadiazole, carbazole, oxazole, oxadiazole and triazole, and metal complexes thereof; and aromatic amine derivatives represented by N,N'-diphenyl-N,N'-di(3-methylphenyl)-4,4'-diphenyl -1,1'-diamine.

Particularly, the dopant to be used when the emissive layer performs triplet emission (phosphorescence emission) is preferably a metal complex compound containing at least one metal selected from the group consisting of iridium (Ir), ruthenium (Ru), palladium (Pd), platinum (Pt), osmium (Os), and rhenium (Re). Preferably, a ligand has a nitrogen-containing aromatic heterocyclic ring such as a phenylpyridine skeleton or a phenylquinoline skeleton. However, the complex is not limited thereto, and a suitable complex is selected in connection with an emitted color, device performance and a host compound to be required. Specific examples thereof include-tris(2-phenylpyridyl) iridium complexes, tris{2-(2-thiophenyl)pyridyl} iridium complexes, tris{2-(2-benzothiophenyl)pyridyl} iridium complexes, tris(2-phenylbenzothiazole) iridium complexes, tris(2-phenylbenzoxazole) iridium complexes, tris-benzoquinoline iridium complexes, bis(2-phenylpyridyl) (acetylacetonato) iridium complexes, bis{2-(2-thiophenyl) pyridyl} iridium complexes, bis{2-(2-benzothiophenyl) pyridyl}(acetylacetonato) iridium complexes, bis(2-phenylbenzothiazole)(acetylacetonato) iridium complexes, bis(2-phenylbenzoxazole) (acetylacetonato) iridium complexes, bisbenzoquinoline(acetylacetonato) iridium complexes, bis{2-(2,4-difluorophenyl)pyridyl} (acetylacetonato) iridium complexes, tetraethylporphyrin platinum complexes, {tris(thenoyltrifluoroacetone) mono(1,10-phenanthroline)} europium complexes, {tris(thenoyltrifluoroacetone)mono(4,7-diphenyl -1,10-phenanthroline)} europium complexes,{tris(1,3-diphenyl -1,3-propanedione)mono(1,10-phenanthroline)} europium complexes and trisacetylacetone terbium complexes. A phosphorescence dopant described in Japanese Patent Laid-open Publication No. 2009-130141 is also preferably used. Although not limited to these, an iridium complex or a platinum complex is preferably used because high luminous efficiency is easily achieved.

Only one of the triplet emissive materials to be used as a dopant material may be contained in an emissive layer, or two or more of the triplet emissive materials may be used in admixture. When two or more triplet emissive materials are used, the total weight of the dopant materials is preferably 30% by weight or less, further preferably 20% by weight or less based on the host material.

The emissive layer may further contain, in addition to the host material and the triplet emissive material, a third component for adjusting the carrier balance in the emissive layer or for stabilizing the layer structure of the emissive layer. It is to be noted that as the third component, a material is selected which does not cause interaction between a host material made of the compound having a carbazole skeleton and represented by the general formula (1) and a dopant material made of a triplet emissive material.

Preferable host and dopant in a triplet emission system are not particularly limited, and specific examples thereof include the following.

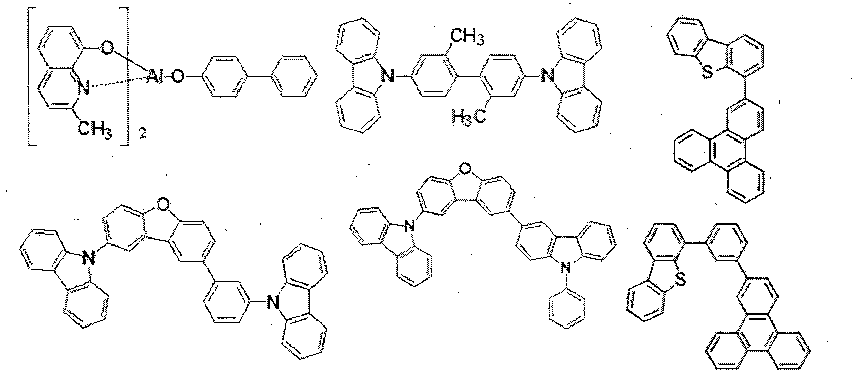

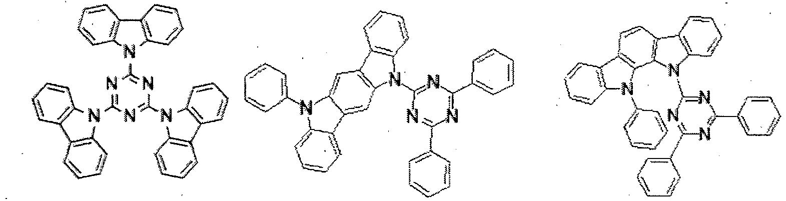

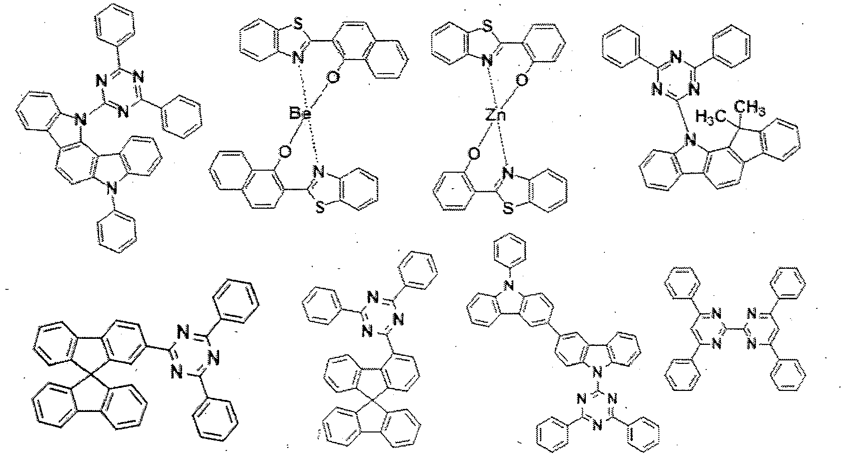

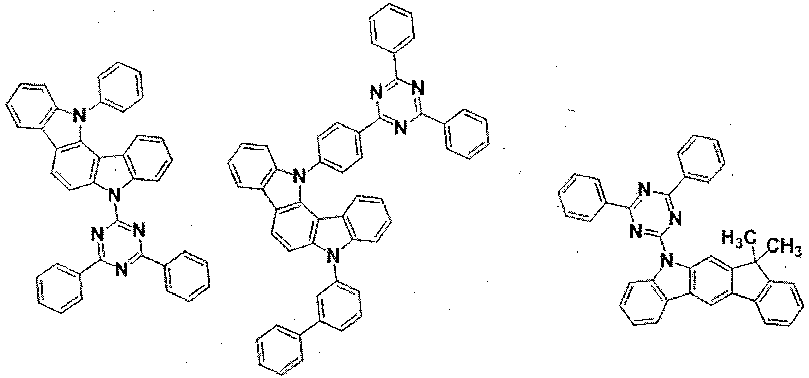

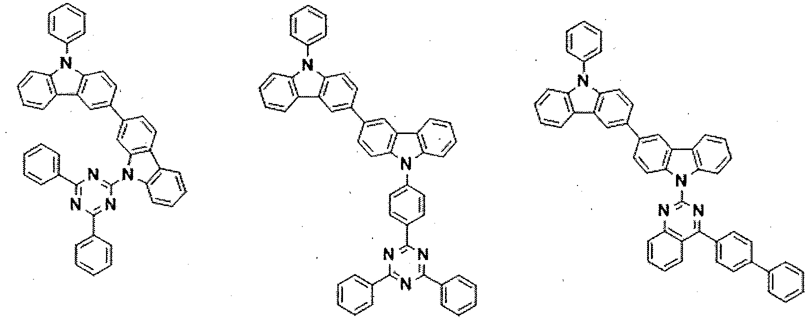

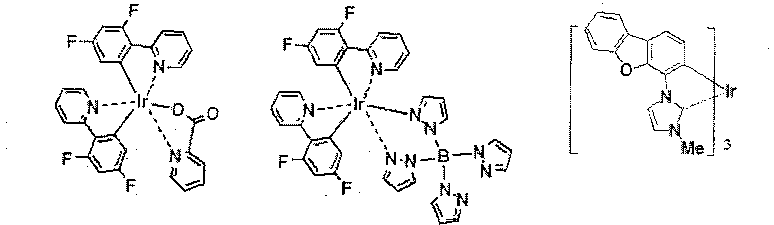

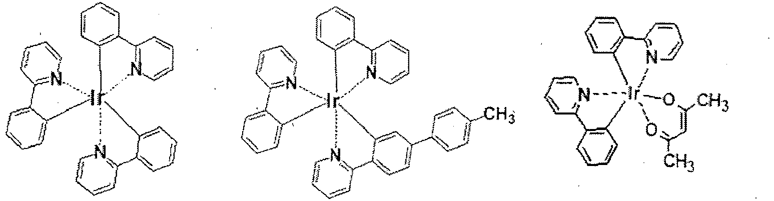

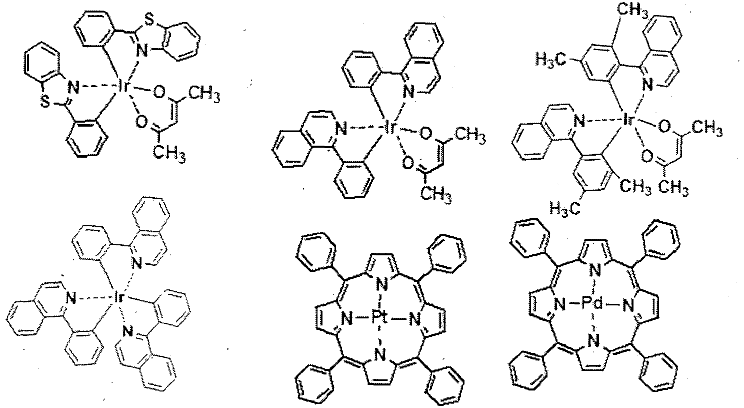

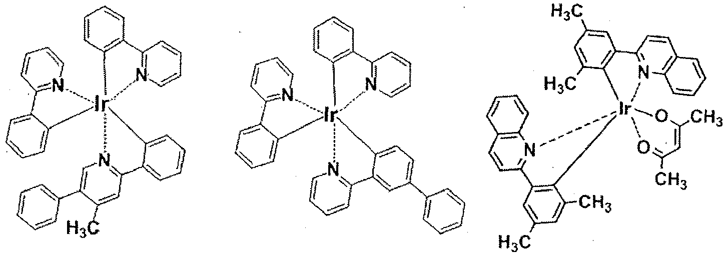

In addition to the examples described above, hosts and dopants may also be used which are disclosed in Appl. Phys. Lett. 78, 1622 (2001), Nature 395, 151 (1998), Appl. Phys. Lett. 90, 123509 (2007), Org. Electron. 1, 15 (2000), US Patent Publication No. 2005-202194, WO 2005-14551, US Patent publication No. 2003-175553, WO 2001-39234, US Patent publication No. 2006-0280965, Appl. Phys. Lett. 77, 2280 (2000), WO 2004-93207, WO 2005-89025, WO 2006-132173, Japanese Patent Laid-open No. 2005-11610, Japanese Patent Laid-open No. 2007-254297, WO 2007-63796, WO 2007-63754, WO 2008-56746, WO 2008-146839, WO 2009-84546, WO 2005-30900, WO 2006-114966, US Patent No. 2006-835469, US Patent publication No. 2006-202194, US Patent publication No. 2007-087321, Adv. Mater. 19, 739 (2007), WO 2003-40257, Chem. Mater. 17, 3532 (2005), Adv. Mater. 17, 1059 (2005), Inorg. Chem. 40, 1704 (2001), US Patent publication No. 2002-034656, US Patent publication No. 2006-687266, Chem. Mater. 16, 2480 (2004), US Patent publication No. 2007-190359, US Patent No. 2006-008670, Japanese Patent Laid-open No. 2007-123392, Adv. Mater. 16, 2003 (2004), Angew. Chem. Int. Ed. 2006, 45, 7800, Appl. Phys. Lett. 86, 153505 (2005), Chem. Lett. 34, 592 (2005), Chem. Commun. 2906 (2005), Inorg. Chem. 42, 1248 (2003), WO 2002-2714, WO 2006-9024, US Patent publication No. 2006-251923, WO 2006-56418, US Patent publication No. 2005-260441, US Patent publication No. 2007-190359, US Patent publication No. 2002-134984, Angew. Chem. Int. Ed. 47, 1 (2008), Chem. Mater. 18, 5119 (2006), Inorg. Chem. 46, 4308 (2007), WO 2005-123873, WO 2007-4380, WO 2006-82742, US Patent publication No. 2005-260449, Organometallics 23, 3745 (2004), Appl. Phys. Lett. 74, 1361 (1999), WO 2006-98120, WO 2006-103874, WO 2012-13271, WO 2011-141109, WO 2011-55934, WO 2011-139055, WO 2011-137072, WO 2011-125680, WO 2011-132684, WO 2011-132683 and the like.

In the present invention, the electron transporting layer is a layer in which electrons are injected from the cathode and, further, which transports the electrons. It is desired that the electron transporting layer has a high electron injection efficiency, and efficiently transports injected electrons. For this reason, the electron transporting layer is required to be constituted by a substance having great electron affinity and, moreover, great electron mobility and, further, excellent stability, and generating impurities that become a trap with difficulty at the time of production and at the time of use. Particularly, when layers are laminated with a large film thickness, a low-molecular weight compound is crystallized or the like to easily degrade film quality, and therefore a compound having a molecular weight of 400 or more, which retains stable film quality, is preferred. However, when transportation balance between holes and electrons is considered, if the electron transporting layer mainly plays a role of being capable of inhibiting holes from the anode from flowing to the cathode side without recombination, even when the layer is constituted by a material having not so high electron transporting ability, the effect of improving luminous efficiency becomes equivalent to that when the layer is constituted by a material having a high electron transporting ability. Therefore, the electron transporting layer in the present invention also includes a hole inhibition layer which can efficiently inhibit the transfer of holes as the same meaning.

Examples of the electron transporting material to be used for the electron transporting layer include fused polycyclic aromatic derivatives, such as naphthalene and anthracene, styryl-based aromatic derivatives typified by 4,4'-bis(diphenylethenyl)biphenyl, quinone derivatives, such as anthraquinone and diphenoquinone, phosphorus oxide derivatives, and various kinds of metal complexes, such as quinolinol complexes, e.g., tris(8-quinolinolato) aluminum(III), benzoquinolinol complexes, hydroxyazole complexes, azomethine complexes, tropolone metal complexes, and flavonol metal complexes. It is preferred to use a compound that includes an element selected from carbon, hydrogen, nitrogen, oxygen, silicon and phosphorus, and has a heteroaryl ring structure containing electron-accepting nitrogen because it can reduce a driving voltage and a highly efficient light emission can be obtained.

The electron-accepting nitrogen referred to herein denotes a nitrogen atom which forms a multiple bond with an adjoining atom. Since nitrogen atoms have high electronegativity, the multiple bond has an electron-accepting nature. Therefore, an aromatic heterocyclic ring containing electron-accepting nitrogen has high electron affinity. An electron transporting material having electron-accepting nitrogen makes easier acceptance of electrons from a cathode having higher electron affinity,'and lower voltage driving becomes possible. In addition, since supply of electrons to an emissive layer is increased and a recombining probability is increased, luminous efficiency is improved.

Examples of the heteroaryl ring containing electron-accepting nitrogen include a pyridine ring, a pyrazine ring, a pyrimidine ring, a quinoline ring, a quinoxaline ring, a naphthyridine ring, a pyrimidopyrimidine ring, a benzoquinoline ring, a phenanthroline ring, an imidazole ring, an oxazole ring, an oxadiazole ring, a triazole ring, a thiazole ring, a thiadiazole ring, a benzoxazole ring, a benzothiazole ring, a benzimidazole ring, and a phenanthroimidazole ring.

Examples of preferred compounds having such a heteroaryl ring structure include benzimidazole derivatives, benzoxazole derivatives, benzothiazole derivatives, oxadiazole derivatives, thiadiazole derivatives, triazole derivatives, pyrazine derivatives, phenanthroline derivatives, quinoxaline derivatives, quinoline derivatives, benzoquinoline derivatives, oligopyridine derivatives such as bipyridine and terpyridine, quinoxaline derivatives and naphthyridine. derivatives. Among them, imidazole derivatives such as tris(N-phenylbenzimidazol-2-yl)benzene; oxadiazole derivatives such as 1,3-bis[(4-tert-butylphenyl) 1,3,4-oxadiazolyl]phenylene; triazole derivatives such as N-naphthyl-2,5-diphenyl-1,3,4-triazole; phenanthroline derivatives such as bathocuproine and 1,3-bis(1,10-phenanthrolin-9-yl)benzene; benzoquinoline derivatives such as 2,2'-bis(benzo[h]quinolin-2-yl)-9,9,'-spirobifluorene; bipyridine derivatives such as 2, 5-bis (6'- (2',2"-bipyridyl)) -1,1-dimethyl-3,4-diphenylsilole; terpyridine derivatives such as 1,3-bis(4'-(2,2':6'2"-terpyridinyl))benzene; and naphthyridine derivatives such as bis(1-naphthyl)-4-(1,8-naphthyridin-2-yl)phenylphosphine oxide are suitably used in view of an electron transporting ability. It is more preferable that such a derivative has a fused polycyclic aromatic skeleton because if so, then the glass transition temperature will increase and an effect of reducing the voltage of a light-emitting device is great due to increased electron mobility. Moreover, considering the improvement in durable life of a device, the easiness of synthesis, and easy availability of raw materials, it is particularly preferable that the fused polycyclic aromatic skeleton is an anthracene skeleton, a pyrene skeleton, or a phenanthroline skeleton. While the electron transporting material may be used alone, two or more kinds of the electron transporting materials may be used in combination, or one or more kinds of other electron transporting materials may be used in combination with the electron transporting material.

Preferable electron transporting materials are not particularly limited, and specific examples thereof include the following.

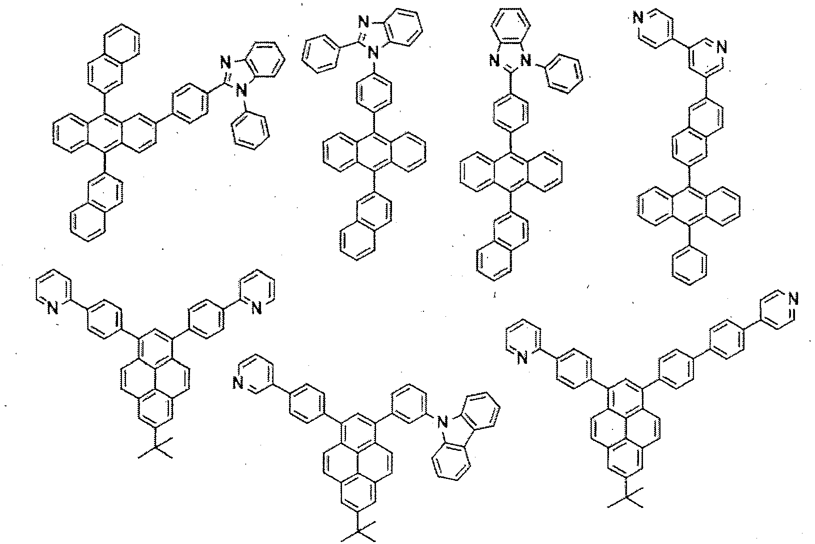

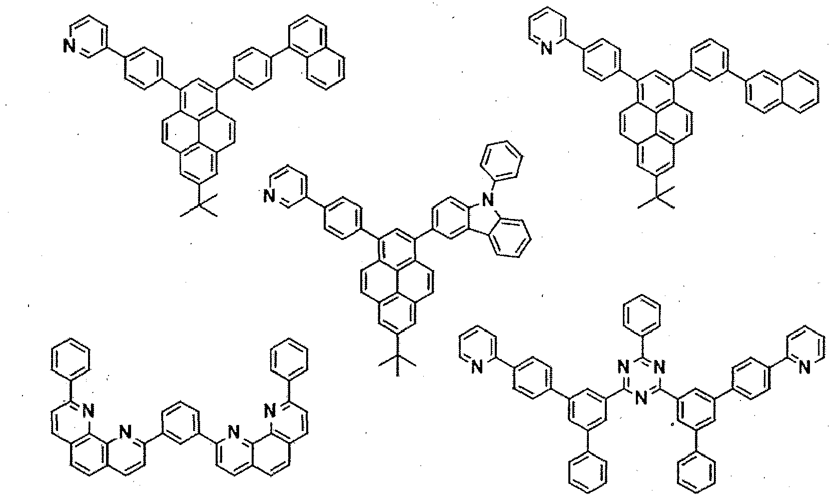

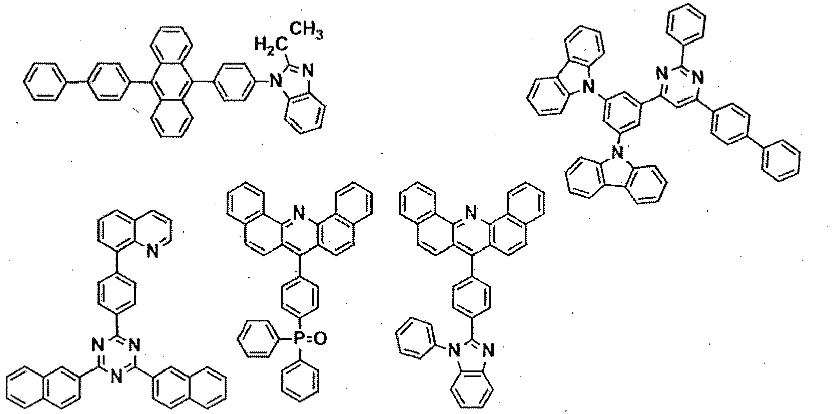

In addition to the examples described above, electron transporting materials may also be used which are disclosed in WO 2004/63159, WO 2003/60956, Appl. Phys. Lett. 74, 865 (1999), Org. Electron. 4, 113 (2003), WO 2010/113743 and WO 2010/1817.

While the electron transporting material may be used alone, two or more kinds of the electron transporting materials may be used in combination, or one or more kinds of other electron transporting materials may be used in combination with the electron transporting material. Moreover, a donor compound may be contained. The donor compound denotes a compound which makes easy electron injection into the electron transporting layer from the cathode or the electron injection layer and, moreover, enhances the electric conductivity of the electron transporting layer, by improving an electron injection barrier.

Preferable examples of the donor compound include an alkali metal, an inorganic salt containing an alkali metal, a complex of an alkali metal and an organic substance, an alkaline earth metal, an inorganic salt containing an alkaline earth metal, or a complex of an alkaline earth metal and an organic substance. Examples of the preferable kind of the alkali metal and the alkaline earth metal include alkali metals such as lithium, sodium, potassium, rubidium and cesium, and alkaline earth metals such as magnesium, calcium, cerium and barium which have a low work function and have a great effect of enhancing electron transporting ability.

In addition, since deposition in vacuum is easy and handling is excellent, the donor compound is preferably in the state of an inorganic salt or a complex with an organic substance rather than a metal single substance. Moreover, from the viewpoints of improvement in easiness in handling in the atmospheric air and easiness in control of the concentration to be added, the donor compound is more preferably in the state of a complex with an organic substance. Examples of the inorganic salt include oxides such as LiO and Li₂O, nitrides, fluorides such as LiF, NaF and KF, and carbonates such as Li₂CO₃, Na₂CO₃, K₂CO₃, Rb₂CO₃ and Cs₂CO₃. Preferable examples of the alkali metal or alkaline earth metal include lithium and cesium from the viewpoint of obtaining a significant low voltage driving effect. In complexes with an organic substance, preferable examples of the organic substance include quinolinol, benzoquinolinol, pyridylphenol, flavonol, hydroxyimidazopyridine, hydroxybenzazole, and hydroxytriazole. Particularly, a complex of an alkali metal and an organic substance is preferred from the viewpoint of a more significant effect of lowering the voltage of the light-emitting device, and further, a complex of lithium and an organic substance is more preferred from the viewpoint of easiness in synthesis and thermal stability, and lithium quinolinol that can be obtained at a relatively low cost is particularly preferred.

The ionization potential of the electron transporting layer is not particularly limited, but is preferably 5.6 eV or more and 8.0 eV or less, more preferably 6.0 eV or more and 7.5 eV or less.

Examples of a method of forming each of the aforementioned layers constituting the light-emitting device include, but are not particularly limited to, resistance heating deposition, electron beam deposition, sputtering, a molecular lamination method, and a coating method, but usually, resistance heating deposition or electron beam deposition is preferable from the viewpoint of device property.

The thickness of the organic layer depends on the resistance value of an emissive substance and, therefore, it cannot be limited, but it is preferably 1 to 1000 nm. The film thickness of each of the emissive layer, the electron transporting layer and the hole transporting layer is preferably 1 nm or more and 200 nm or less, more preferably 5 nm or more and 100 nm or less.

The light-emitting device of the present invention has a function of being capable of converting electric energy into light. Herein, a direct current is mainly used as the electric energy, but a pulse current or an alternate current can also be used. A current value and a voltage value are not particularly limited, but when the power consumed and life of the device are considered, they should be selected so that the maximum luminance is obtained by energy as low as possible.

The light-emitting device of the present invention is used suitably as a display that displays in a matrix and/or segment system.

In the matrix system, pixels for display are arranged two-dimensionally such as lattice-like arrangement or mosaic-like arrangement, and the collection of pixels displays letters and images. The shape and size of the pixel are determined depending on utility. For example, for displaying images and letters on personal computers, monitors and televisions, a square pixel being 300 µm or less at each side is usually used and, in the case of a large display such as a display panel, a pixel being millimeter order at each side is used. In the case of a monochromatic display, pixels having the same color may be arranged, and in the case of a color display, pixels having red, green and blue colors are arranged to perform display. In this case, typically, there are a delta type and a stripe type. A method of driving this matrix may be either a passive matrix driving method or an active- matrix. The passive matrix driving has a simple structure, but when an operation property is considered, the active matrix is more excellent in some cases, and it is necessary to use them properly depending on utility.

The segment system in the present invention is a system by which a pattern is formed so as to display predetermined information, and a region determined by arrangement of this pattern is made to emit light. Examples thereof include time and temperature displays in digital watches and thermometers, operating-state displays in audio equipment, IH cookers and so on, and panel displays of automobiles. The above-mentioned matrix display and segment display may exist together in the same panel.

The light-emitting device of the present invention can also be preferably used as backlight of various instruments. Backlight is used mainly for the purpose of enhancing the visibility of display apparatuses which do not emit light by themselves, and is used in liquid crystal display equipment, clocks, audio equipment, automobile panels, display panels, signs, and the like. In particular, the light-emitting device of the present invention is preferably used in backlight for liquid crystal display apparatuses, inter alia, for personal computers which are studied to be thinned, and can provide backlight thinner and lighter than conventional products.

### EXAMPLES

The present invention will be described by way of Examples, but the present invention is not limited thereto. The number of the compound in each of Examples described below indicates the number of the compound described above.

### Synthesis Example 1.

### Synthesis of Compound [1]

A mixed,solution of 20.9 g of 3-bromocarbazole, 15.0 g of phenylcarbazole-3-boronic acid, 366 mg of palladium acetate, 300 mg of tris (2-methylphenyl)phosphine, 105 ml of a 2 M aqueous potassium carbonate solution and 260 ml of dimethoxyethane was refluxed for 6 hours under a nitrogen flow. The solution was cooled to room temperature, and then extracted with 500 ml of toluene. The organic layer was washed with 100 ml of water twice, dried over magnesium sulfate, and then evaporated. The resultant concentrate was purified by silica gel column chromatography, and vacuum-dried to obtain 13.5 g of 9-phenyl-9H,9'H-3,3'-bicarbazole.

Next, a mixed solution of 4.0 g of 4-bromobiphenyl, 3.2 g of 3-chlorophenylboronic acid, 12 mg of bis(triphenylphosphine) palladium(II) dichloride, 21 ml of a 2.0 M aqueous sodium carbonate solution and 40 ml of dimethoxyethane was refluxed for 4 hours under a nitrogen flow. The solution was cooled to room temperature, and then extracted with 100 ml of toluene. The organic layer was washed with 50 ml of water three times, dried over magnesium sulfate, and then evaporated. The resultant concentrate was purified by silica gel column chromatography, and vacuum-dried to obtain 3.7 g of 3-chloro-1,1':4',1"-terphenyl.

Next, a mixed solution of 2.5 g of 9-phenyl-9H,9'H-3,3'-bicarbazole, 1.9 g of 3-chloro-1,1':4',1"-terphenyl, 35 mg of bis(dibenzylideneacetone) palladium, 43 mg of cBRIDP, 820 mg of sodium tert-butoxide and 30 ml of o-xylene was heated and stirred for 3 hours under reflux under a nitrogen flow. The solution was cooled to room temperature, and then extracted with 100 ml of toluene. The organic layer was washed with 50 ml of water three times, dried over magnesium sulfate, and then evaporated. The resultant concentrate was purified by silica gel column chromatography, and evaporated, and the solid thus obtained was vacuum-dried to obtain 2.8 g of a white solid.

¹H-NMR analytical results of the resultant powder are as follows, and it was confirmed that the resultant white solid was the compound [1].
¹H-NMR (CDCl₃ (d=ppm)):7.29-7.82 (m,27H), 7.91 (d, 1H), 8.23-8.28 (m,2H), 8.47-8.48 (t,2H)
The compound [1] was used as a light-emitting device material after sublimation purification was performed at about 330°C under a pressure of 1 × 10⁻³ Pa using an oil diffusion pump. The HPLC purity (area % at a measurement wavelength of 254 nm) was 99.8% before sublimation purification, and 99.9% after sublimation purification.

### Synthesis Example 2

### Synthesis of Compound [18]

A mixed solution of 20.9 g of 3-bromocarbazole, 15.0 g of phenylcarbazole-3-boronic acid, 366 mg of palladium acetate, 300 mg of tris (2-methylphenyl)phosphine, 105 ml of a 2 M aqueous potassium carbonate solution and 260 ml of dimethoxyethane was refluxed for 6 hours under a nitrogen flow. The solution was cooled to room temperature, and then extracted with 500 ml of toluene. The organic layer was washed with 100 ml of water twice, dried over magnesium sulfate, and then evaporated. The resultant concentrate was purified by silica gel column chromatography, and vacuum-dried to obtain 13.5 g of 9-phenyl-9H,9'H-3,3'-bicarbazole.

Next, a mixed solution of 4.0 g of 4-bromochlorobenzene, 5.0 g of 3-biphenylboronic acid, 15 mg of
bis(triphenylphosphine) palladium(II) dichloride, 25 ml of a 2.0 M aqueous sodium carbonate solution and 50 ml of dimethoxyethane was refluxed for 4 hours under a nitrogen flow. The solution was cooled to room temperature, and then extracted with 100 ml of toluene. The organic layer was washed with 50 ml of water three times, dried over magnesium sulfate, and then evaporated. The resultant concentrate was purified by silica gel column chromatography, and vacuum-dried to obtain 4.11 g of 4-chloro-1,1':3',1"-terphenyl.

Next, a mixed solution of 2.5 g of 9-phenyl-9H,9'H-3,3'-bicarbazole, 1.9 g of 4-chloro-1,1':3',1"-terphenyl, 35 mg of bis (dibenzylideneacetone) palladium, 43 mg of cBRIDP, 820 mg of sodium tert-butoxide and 30 ml of o-xylene was heated and stirred for 3 hours under reflux under a nitrogen flow. The solution was cooled to room temperature, and then extracted with 100 ml of toluene. The organic layer was washed with 50 ml of water three times, dried over magnesium sulfate, and then evaporated. The resultant concentrate was purified by silica gel column chromatography, and evaporated, and the solid thus obtained was vacuum-dried to obtain 2.4 g of a white solid.

¹H-NMR analytical results of the resultant powder are as follows, and it was confirmed that the resultant white solid was the compound [18].
¹H-NMR (CDCl₃ (d=ppm)):7.29-7.83 (m,25H), 7.89-7.94 (m,3H),
The compound [18] was used as a light-emitting device material after sublimation purification was performed at about 330°C under a pressure of 1 × 10⁻³ Pa using an oil diffusion pump. The HPLC purity (area % at a measurement wavelength of 254 nm) was 99.6% before sublimation purification, and 99.9% after sublimation purification.

### Synthesis Example 3

### Synthesis of Compound [19] (not forming part of the present invention)

A mixed solution of 20.9 g of 3-bromocarbazole, 15.0 g of phenylcarbazole-3-boronic acid, 366 mg of palladium acetate, 300 mg of tris (2-methylphenyl)phosphine, 105 ml of a 2 M aqueous potassium carbonate solution and 260 ml of dimethoxyethane was refluxed for 6 hours under a nitrogen flow. The solution was cooled to room temperature, and then extracted with 500 ml of toluene. The organic layer was washed with 100 ml of water twice, dried over magnesium sulfate, and then evaporated. The resultant concentrate was purified by silica gel column chromatography, and vacuum-dried to obtain 13.5 g of 9-phenyl-9H,9'H-3,3'-bicarbazole.

Next, a mixed solution of 4.0 g of 4-bromochlorobenzene, 5.0 g of 4-biphenylboronic acid, 15 mg of bis(triphenylphosphine) palladium(II) dichloride, 25 ml of a 2.0 M aqueous sodium carbonate solution and 50 ml of dimethoxyethane was refluxed for 4 hours under a nitrogen flow. The solution was cooled to room temperature, and then extracted with 100 ml of toluene. The organic layer was washed with 50 ml of water three times, dried over magnesium sulfate, and then evaporated. The resultant concentrate was purified by silica gel column chromatography, and vacuum-dried to obtain 4.7 g of 4-chloro-1,1':4',1"-terphenyl.

Next, a mixed solution of 2.5 g of 9-phenyl-9H,9'H-3,3'-bicarbazole, 1.9 g of 4-chloro-1,1':4',1"-terphenyl, 35 mg of bis(dibenzylideneacetone) palladium, 43 mg of cBRIDP, 820 mg of sodium tert-butoxide and 30 ml of o-xylene was heated and stirred for 3 hours under reflux under a nitrogen flow. The mixture was cooled to room temperature, and then filtered, 100 ml of pure water was added to the resultant solid, and the mixture was stirred for 1 hour, and then filtered. The resultant solid was dissolved in 100 ml of tetrahydrofuran, 300 mg of activated carbon was then added, and the mixture was stirred for 1 hour, filtered, and then evaporated. The resultant solid was vacuum-dried to obtain 1.6 g of a white solid.

¹H-NMR analytical results of the resultant powder are as follows, and it was confirmed that the resultant white solid was the compound [19].
¹H-NMR (CDCl₃ (d=ppm)) :7.30-7.83 (m, 26H), 7.89-7.92 (d, 2H), 8.24-8.28 (m, 2H), 8.47-8.48 (d, 2H)

The compound [19] was used as a light-emitting device material after sublimation purification was performed at about 340°C under a pressure of 1 × 10⁻³ Pa using an oil diffusion pump. The HPLC purity (area % at a measurement wavelength of 254 nm) was 99.8% before sublimation purification, and 99.9% after sublimation purification.

### Synthesis Example 4

### Synthesis of Compound [21]

A mixed solution of 20.9 g of 3-bromocarbazole, 15.0 g of 9-phenylcarbazole-3-boronic acid, 366 mg of palladium acetate, 300 mg of tris (2-methylphenyl)phosphine, 105 ml of a 2M aqueous potassium carbonate solution and 260 ml of dimethoxyethane was refluxed for 6 hours under a nitrogen flow. The solution was cooled to room temperature, and then extracted with 500 ml of tetrahydrofuran. The organic layer was washed with 100 ml of a saturated saline solution twice, dried over magnesium sulfate, and then evaporated. The resultant concentrate was purified by o-xylene recrystallization, and vacuum-dried to obtain 13.5 g of 9-phenyl-9H,9'H-3,3'-bicarbazole.

Next, a mixed solution of 2.9 g of 4-bromochlorobenzene, 5.0 g of 3,5-diphenylbenzeneboronic acid, 11 mg of bis(triphenylphosphine) palladium(II) dichloride, 18 ml of a 2.0 M aqueous sodium carbonate solution and 40 ml of dimethoxyethane was refluxed for 5 hours under a nitrogen flow. The solution was cooled to room temperature, and then extracted with 100 ml of toluene. The organic layer was washed with 50 ml of water three times, dried over magnesium sulfate, and then evaporated. The resultant concentrate was purified by silica gel column chromatography, and vacuum-dried to obtain 3.34 g of 4-chloro-5'-phenyl-1,1':3',1"-terphenyl.

Next, a mixed solution of 2.5 g of 9-phenyl-9H,9'H-3,3'-bicarbazole, 2.5 g of 4-chloro-5'-phenyl-1,13',1"-terphenyl, 35 mg of bis(dibenzylideneacetone) palladium, 43 mg of cBRIDP, 820 mg of sodium tert-butoxide and 30 ml of o-xylene was heated and stirred for 3 hours under reflux under a nitrogen flow. The solution was cooled to room temperature, and then extracted with 100 ml of toluene. The organic layer was washed with 50 ml of water three times, dried over magnesium sulfate, and then evaporated. The resultant concentrate was purified by silica gel column chromatography, and evaporated, and the solid thus obtained was vacuum-dried to obtain 2.4 g of a white solid.

¹H-NMR analytical results of the resultant powder are as follows, and it was confirmed that the resultant white solid was the compound [21].
¹H-NMR (CDCl₃ (d=ppm)):7.29-7.68 (m, 19H), 7.73-7.99 (m, 13H), 8.24-8.28 (m,2H), 8.48 (s,2H)
The compound [21] was used as a light-emitting device material after sublimation purification was performed at about 340°C under a pressure of 1 × 10⁻³ Pa using an oil diffusion pump. The HPLC purity (area % at a measurement wavelength of 25.4 nm) was 99.7% before sublimation purification, and 99.9% after sublimation purification.

### Synthesis Example 5

### Synthesis of Compound [24]

A mixed solution of 20.9 g of 3-bromocarbazole, 15.0 g of phenylcarbazole-3-boronic acid, 366 mg of palladium acetate, 300 mg of tris (2-methylphenyl)phosphine, 105 ml of a 2 M aqueous - potassium carbonate solution and 260 ml of dimethoxyethane was refluxed for 6 hours under a nitrogen flow. The solution was cooled to room temperature, and then extracted with 500 ml of toluene. The organic layer was washed with 100 ml of water twice, dried over magnesium sulfate, and then evaporated. The resultant concentrate was purified by silica gel column chromatography, and vacuum-dried to obtain 13.5 g of 9-phenyl-9H,9'H-3,3'-bicarbazole.

Next, a mixed solution of 4.6 g of 4-bromochlorobenzene, 5.0 g of 1-naphtylboronic acid, 17 mg of bis(triphenylphosphine) palladium(II) dichloride, 29 ml of a 2.0 M aqueous sodium carbonate solution and 60 ml of dimethoxyethane was refluxed for 4 hours under a nitrogen flow. The solution was cooled to room temperature, and then extracted with 150 ml of toluene. The organic layer was washed with 80 ml of water three times, dried over magnesium sulfate, and then evaporated. The resultant concentrate was purified by silica gel column chromatography, and vacuum-dried to obtain 4.5 g of 1-(4-chlorophenyl)naphthalene.

Next, a mixed solution of 2.5 g of 9-phenyl-9H,9'H-3,3'-bicarbazole, 1.8 g of 1-(4-chlorophenyl)naphthalene, 35 mg of bis(dibenzylideneacetone) palladium, 43 mg of cBRIDP, 820 mg of sodium tert-butoxide and 30 ml of o-xylene was heated and stirred for 3 hours under reflux under a nitrogen flow. The solution was cooled to room temperature, and then extracted with 100 ml of toluene. The organic layer was washed with 50 ml of water three times, dried over magnesium sulfate, and then evaporated. The resultant concentrate was purified by silica gel column chromatography, and evaporated, and the solid thus obtained was vacuum-dried to obtain 3.1 g of a white solid.

¹H-NMR analytical results of the resultant powder are as follows, and it was confirmed that the resultant white solid was the compound [24].
¹H-NMR (CDCl₃ (d=ppm)):7.30-7.69 (m,17H), 7.78-7.86 (m,6H), 7.92-7.99 (m,2H), 8.06-8.10 (t,1H), 8.24-8.29 (m,2H), 8.48-8.50 (t,2H)
The compound [24] was used as a light-emitting device material after sublimation purification was performed at about 330°C under a pressure of 1 × 10⁻³Pa using an oil diffusion pump. The HPLC purity (area % at a measurement wavelength of 254 nm) was 99.6% before sublimation purification, and 99.9% after sublimation purification.

### Synthesis Example 6

### Synthesis of Compound [4]

A white solid was obtained by performing synthesis in the same manner as in Synthesis Example 2 except that (3,5-diphenylphenyl)boronic acid was used in place of 3-biphenylboronic acid. ¹H-NMR analytical results of the resultant powder are as follows, and it was confirmed that the resultant white solid was the compound [4].
¹H-NMR (DMSO-d₆ (d=ppm)):7.29-7.60 (m,20H), 7.65-7.74 (m,7H), 7.80-7.93 (m,10H), 8.02-8.08 (m,4H), 8.22 (s,1H), 8.39 (t,2H, J=6.8Hz), 8.69 (dd,2H,¹J=4.3Hz,²J=1.6Hz).

The compound [4] was used as a light-emitting device material after sublimation purification was performed at about 350°C under a pressure of 1 × 10⁻³ Pa using an oil diffusion pump. The HPLC purity (area % at a measurement wavelength of 254 nm) was 99.7% before sublimation purification, and 99.9% after sublimation purification.

### Synthesis Example 7

### Synthesis of Compound [17]

A white solid was obtained by performing synthesis in the same manner as in Synthesis Example 2 except that 2-biphenylboronic acid was used in place of 3-biphenylboronic acid. ¹H-NMR analytical results of the resultant powder are as follows, and it was confirmed that the resultant white solid was the compound [17].
¹H-NMR (DMSO-d₆ (d=ppm)):7.20-7.75 (m,26H), 7.88 (dt,2H,¹J=8.6Hz,²J=1.8Hz), 8.38(dd,2H,¹J=7.7Hz,²J=2.6Hz), 8.68(dd,2H,¹J=3.2Hz,²J=1.6Hz).

The compound [17] was used as a light-emitting device material after sublimation purification was performed at about 320°C under a pressure of 1 × 10⁻³ Pa using an oil diffusion pump. The HPLC purity (area % at a measurement wavelength of 254 nm) was 99.7% before sublimation purification, and 99.9% after sublimation purification.

### Synthesis Example 8

### Synthesis of Compound [7]

A white solid was obtained by performing synthesis in the same manner as in Synthesis Example 2 except that 2-naphthaleneboronic acid was used in place of 3-biphenylboronic acid. ¹H-NMR analytical results of the resultant powder are as follows, and it was confirmed that the resultant white solid was the compound [7]. ¹H-NMR (DMSO-d₆ (d=ppm)):7.30-7.74 (m,15H), 7.82-8.11 (m,10H), 8.40 (m,3H), 8.70 (dd,2H,¹J=4.1Hz, ²J=1.6Hz).

The compound [7] was used as a light-emitting device material after sublimation purification was performed at about 320°C under a pressure of 1 × 10⁻³ Pa using an oil diffusion pump. The HPLC purity (area % at a measurement wavelength of 254 nm) was 99.7% before sublimation purification, and 99.9% after sublimation purification.

### Example 1

A glass substrate with an ITO transparent electroconductive film deposited thereon in a thickness of 50 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of a device, and placed in a vacuum deposition apparatus, and the air was evacuated until the degree of vacuum in the apparatus was 5 × 10⁻⁴ Pa or lower. By a resistance heating method, HI-1 was deposited as a hole injection layer in a thickness of 10 nm. Next, HT-1 was deposited as a first hole transporting layer in a thickness of 110 nm. Next, a compound [1] was deposited as a second hole transporting layer in a thickness of 10 nm. Next, a compound H-1 and a compound D-1 were used as a host material and as a dopant material, respectively, and were deposited as an emissive layer in a thickness of 40 nm so that the doping concentration of the dopant material was 5% by weight. Next, as an electron transporting layer, a compound E-1 was laminated in a thickness of 20 nm.

Next, lithium fluoride was deposited in a thickness of 0.5 nm, and aluminum was deposited in a thickness of 60 nm to form a cathode, so that a 5 × 5 mm square device was prepared. The film thickness referred to herein is an indicated value on a crystal oscillation film thickness monitor. When the light-emitting device was driven at a direct current of 10 mA/cm², blue light was emitted with a luminous efficiency of 5.1 lm/W. When the light-emitting device was continuously driven at a direct current of 10 mA/cm², the luminance decreased by half in 1500 hours. Compounds HI-1, HT-1, H-1, D-1 and E-1 are the compounds shown below.

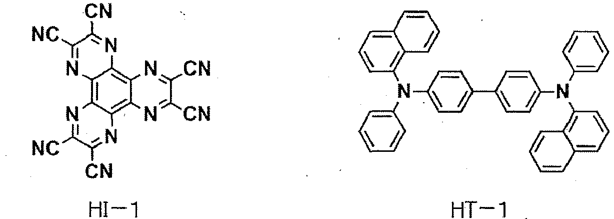

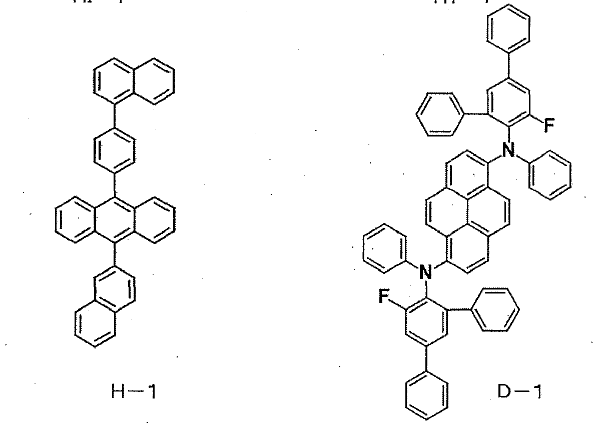

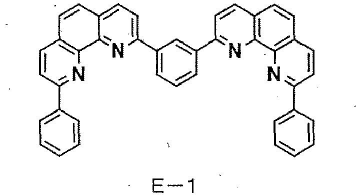

### (Examples 3 and 7-10 using compounds 19, 59, 68, 138 and 149 are not forming part of the present invention)

Light-emitting devices were prepared in the same manner as in Example 1 except that materials described in Table 1 were used as a second hole transporting layer. The results of the examples are shown in Table 1.

### Comparative Examples 1 to 8

In the same manner as in Example 1 except that materials described in Table 1 were as a second hole transporting layer, light-emitting devices were prepared and evaluated. The results are shown in Table 1. HT-2 to HT-9 are the compounds shown below.

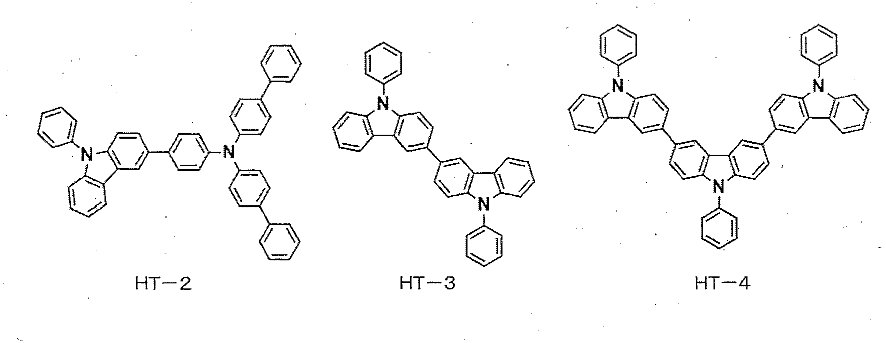

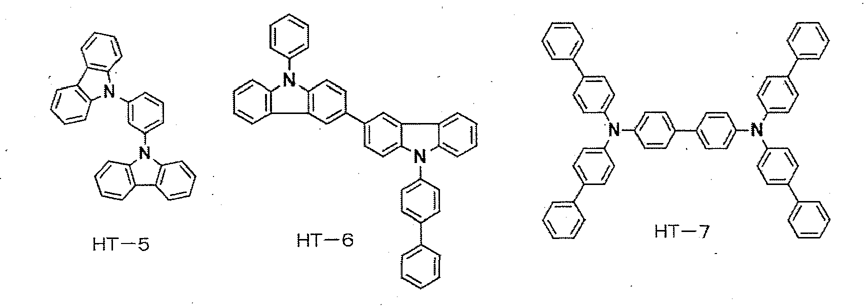

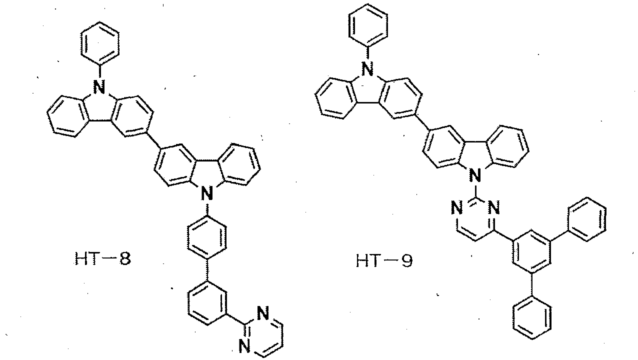

### Example 11

A glass substrate with an ITO transparent electroconductive film deposited thereon in a thickness of 50 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of a device, and placed in a vacuum deposition apparatus, and the air was evacuated until the degree of vacuum in the apparatus was 5 × 10⁻⁴ Pa or lower. By a resistance heating method, HI-1 was deposited as a hole injection layer in a thickness of 10 nm. Next, HT-1 was deposited as a first hole transporting layer in a thickness of 110 nm. Next, a compound [1] was deposited as a second hole transporting layer in a thickness of 10 nm. Next, a compound H-2 and a compound D-2 were used as a host material and as a dopant material, respectively, and were deposited as an emissive layer in a thickness of 40 nm so that the doping concentration of the dopant material was 10% by weight. Next, a layer obtained by mixing an organic compound (E-2) and a donor compound (lithium quinolinol) at a deposition speed ratio of 1 : 1 (= 0.05 nm/s : 0.05 nm/s) was laminated as an electron transporting layer in a thickness of 10 nm.

Next, lithium quinolinol was deposited in a thickness of 1 nm, and a co-deposited film of magnesium and silver was deposited in a thickness of 100 nm at a deposition speed ratio of magnesium : silver = 10 : 1 (= 0.5 nm/s : 0.05 nm/s) to form a cathode, so that a 5 × 5 mm square device was prepared. The film thickness referred to herein is an indicated value on a crystal oscillation film thickness monitor. When the light-emitting device was driven at a direct current of 10 mA/cm², green light was emitted with a luminous efficiency of 14 . 0 lm/W. When the light-emitting device was continuously driven at a direct current of 10 mA/cm², the luminance decreased by half in 1400 hours. H-2, D-2 and E-2 are the compounds shown below.

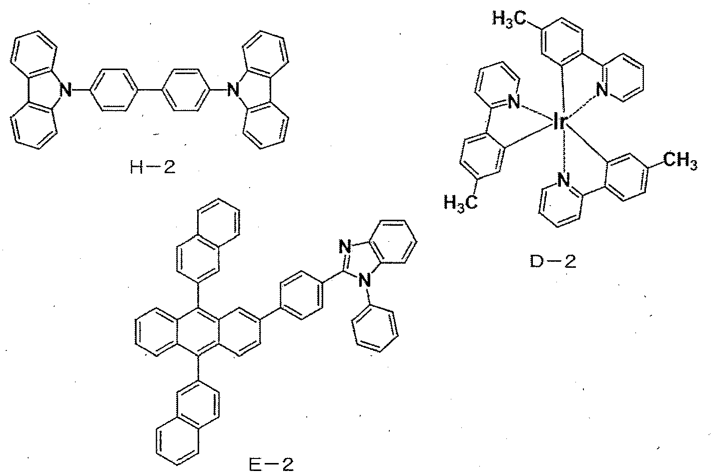

### Examples 12 to 19

### (Examples 13, 18 and 19 using compounds 19, 113 and 173 are not forming part of the present invention)

In the same manner as in Example 11 except that materials described in Table 2 were used as a second hole transporting layer, a host material and a dopant material, light-emitting devices were prepared and evaluated. The results are shown in Table 2.

### Comparative Examples 9 to 15

In the same manner as in Example 11 except that compounds described in Table 2 were used as a second hole transporting layer, a host material and a dopant material, light-emitting devices were prepared and evaluated. The results are shown in Table 2. HT-10 is the compound shown below.

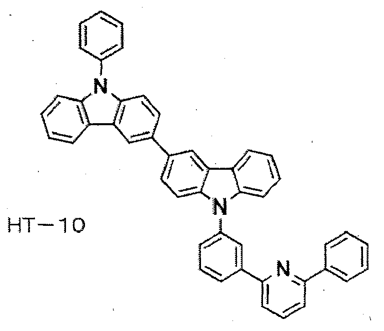

### Example 20

A glass substrate with an ITO transparent electroconductive film deposited thereon in a thickness of 50 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of a device, and placed in a vacuum deposition apparatus, and the air was evacuated until the degree of vacuum in the apparatus was 5 × 10⁻⁴ Pa or lower. By a resistance heating method, HI-1 was deposited as a hole injection layer in a thickness of 10 nm. Next, HT-1 was deposited as a hole transporting layer in a thickness of 125 nm. Next, a compound [1] and a compound D-2 were used as a host material and as a dopant material, respectively, and were deposited as an emissive layer in a thickness of 40 nm so that the doping concentration of the dopant material was 10% by weight. Next, as an electron transporting layer, a compound E-1 was laminated in a thickness of 20 nm.

Next, lithium fluoride was deposited in a thickness of 0.5 nm, and aluminum was deposited in a thickness of 60 nm to form a cathode, so that a 5 × 5 mm square device was prepared. The film thickness referred to herein is an indicated value on a crystal oscillation film thickness monitor. When the light-emitting device was driven at a direct current of 10 mA/cm², green light was emitted with a luminous efficiency of 17.2 lm/W. When the light-emitting device was continuously driven at a direct current of 10 mA/cm², the luminance decreased by half in 1400 hours.

### (Examples 23 and 24 using compounds 88 and 170 are not forming part of the present invention)

In the same manner as in Example 20 except that materials described in Table 3 were used as a hole transporting layer, a host material and a dopant material, light-emitting devices were prepared and evaluated. The results are shown in Table 3.

### Comparative Examples 16 to 24

In the same manner as in Example 20 except that compounds described in Table 3 were used as a hole transporting layer, a host material and a dopant material, light-emitting devices were prepared and evaluated. The results are shown in Table 3. Compounds H-3, H-4, H-5 and H-6 are the compounds shown below.

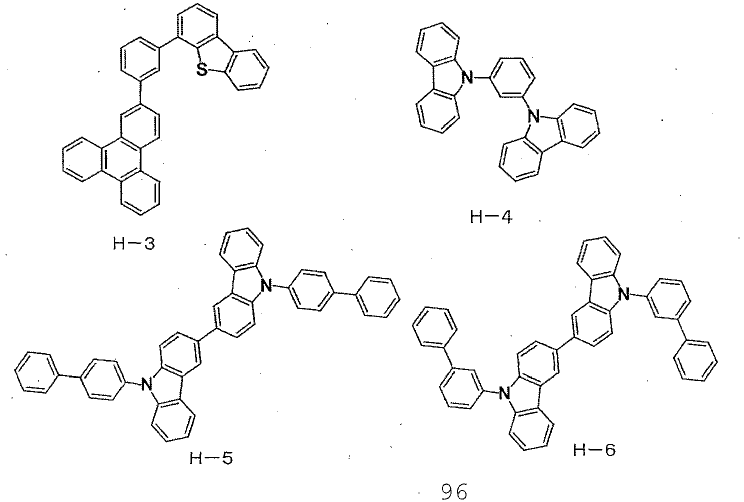

### Example 25

A glass substrate with an ITO transparent electroconductive film deposited thereon in a thickness of 50 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of a device, and placed in a vacuum deposition apparatus, and the air was evacuated until the degree of vacuum in the apparatus was 5 × 10⁻⁴ Pa or lower. By a resistance heating method, HI-1 was deposited as a hole injection layer in a thickness of 10 nm. Next, HT-7 was deposited as a first hole transporting layer in a thickness of 90 nm. Next, a compound [1] was deposited as a second hole transporting layer in a thickness of 30 nm. Next, a compound H-7 and a compound D-3 were used as a host material and as a dopant material, respectively, and were deposited as an emissive layer in a thickness of 30 nm so that the doping concentration of the dopant material was 4% by weight. Next, as an electron transporting layer, a compound E-1 was laminated in a thickness of 35 nm.

Next, lithium fluoride was deposited in a thickness of 0.5 nm, and aluminum was deposited in a thickness of 60 nm to form a cathode, so that a 5 × 5 mm square device was prepared. The film thickness referred to herein is an indicated value on a crystal oscillation film thickness monitor. When the light-emitting device was driven at a direct current of 10 mA/cm², red light was emitted with a luminous efficiency of 10.5 lm/W. When the light-emitting device was continuously driven at a direct current of 10 mA/cm², the luminance decreased by half in 1400 hours. Compounds H-7 and D-3 are the compounds shown below.

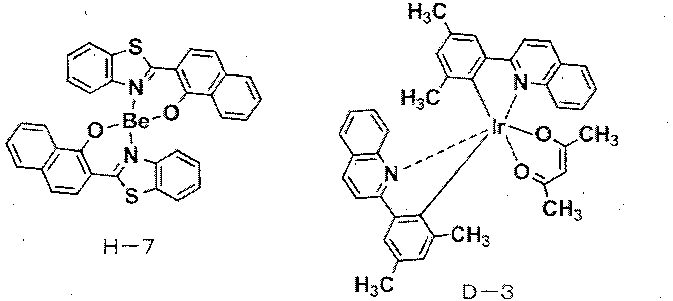

### Examples 26 to 32

### (Examples 27 and 32 using compounds 19 and 57 are not forming part of the present invention)

In the same manner as in Example 25 except that materials described in Table 4 were used as a second hole transporting layer, a host material and a dopant material, light-emitting devices were prepared and evaluated. The results are shown in Table 4.

### Comparative Examples 25 to 30

In the same manner as in Example 25 except that compounds described in Table 4 were used as a second hole transporting layer, a host material and a dopant material, light-emitting devices were prepared and evaluated. The results are shown in Table 4. HT-11 is the compound shown below.

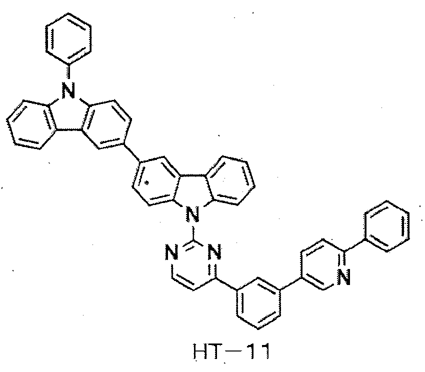

### Example 33

A glass substrate with an ITO transparent electroconductive film deposited thereon in a thickness of 50 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of a device, and placed in a vacuum deposition apparatus, and the air was evacuated until the degree of vacuum in the apparatus was 5 × 10⁻⁴ Pa or lower. By a resistance heating method, HI-1 was deposited as a hole injection layer in a thickness of 10 nm. Next, HT-8 was deposited as a first hole transporting layer in a thickness of 80 nm. Next, a compound [1] was deposited as a second hole transporting layer in a thickness of 10 nm. Next, a compound H-8 and a compound D-4 were used as a host material and as a dopant material, respectively, and were deposited as an emissive layer in a thickness of 30 nm so that the doping concentration of the dopant material was 10% by weight. Next, a layer obtained by mixing an organic compound (E-2) and a donor compound (Liq: lithium quinolinol) at a deposition speed ratio of 1 : 1 (= 0.05 nm/s : 0.05 nm/s) was laminated as an electron transporting layer in a thickness of 35 nm.

Next, lithium quinolinol was deposited in a thickness of 1 nm, and a co-deposited film of magnesium and silver was deposited in a thickness of 100 nm at a deposition speed ratio of magnesium : silver = 10 : 1 (= 0.5 nm/s : 0.05 nm/s) to form a cathode, so that a 5 × 5 mm square device was prepared. The film thickness referred to herein is an indicated value on a crystal oscillation film thickness monitor. When the light-emitting device was driven at a direct current of 10 mA/cm², green light was emitted with a luminous efficiency of 46.0 lm/W. When the light-emitting device was continuously driven at a direct current of 10 mA/cm², the luminance decreased by half in 4500 hours. H-8 and D-4 are the compounds shown below.

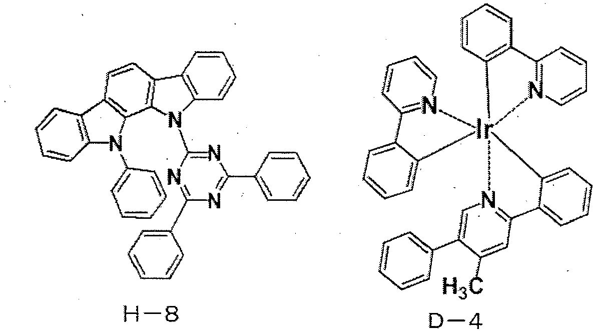

### Examples 34 to 39

### (Examples 38 and 39 using compounds 44 and 62 are not forming part the present invention)

In the same manner as in Example 33 except that materials described in Table 5 were used as a second hole transporting layer, light-emitting devices were prepared and evaluated. The results are shown in Table 5.

### Comparative Examples 31 to 37

In the same manner as in Example 33 except that compounds described in Table 5 were used as a second hole transporting layer, light-emitting devices were prepared and evaluated. The results are shown in Table 5. HT-12 is the compound shown below.

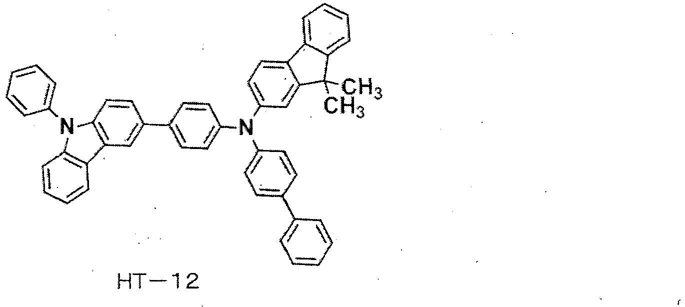

### Examples 40 to 45

### (Examples 44 and 45 using compounds 100 and 149 are not forming part of the present invention)

Light-emitting devices were prepared in the same manner as in Example 33 except that a compound HT-12 and a compound HI-2 were used in place of the compound HI-1, and were deposited as a hole injection layer in a thickness of 10 nm so that the doping concentration of the compound HI-2 was 5% by weight based on the compound HT-12. The results are shown in Table 6. HI-2 is the compound shown below.

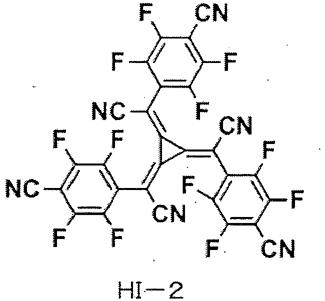

### Examples 46 to 51

### (Example 51 using compound 78 is not forming part of the present invention)

Light-emitting devices were prepared in the same manner as in Examples 40 to 45 except that as a host material, a mixed host of a compound H-8 and a compound H-9 (formed by depositing a co-deposited film of the compound H-8 and the compound H-9 at a deposition speed ratio of 1 : 1, and further depositing a dopant) was used in place of the compound H-8. The results are shown in Table 6. H-9 is the compound shown below.

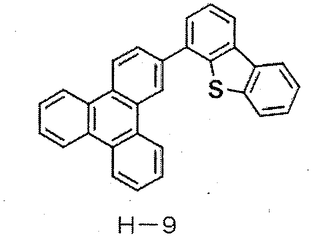

### Examples 52 to 57

### (Examples 56 and 57 using compounds 90 and 117 are not forming part of the present invention)

Light-emitting devices were prepared in the same manner as in Examples 33 to 39 except that as a hole injection layer, a compound HI-3 was used in place of the compound HI-1. The results are shown in Table 6. HI-3 is the compound shown below.

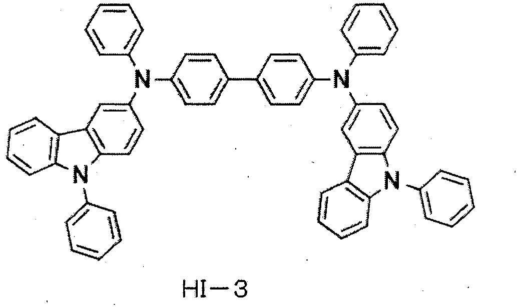

### Examples 58 to 62

Light-emitting devices were prepared in the same manner as in Example 28 except that as an electron transporting layer, materials described in Table 6 were used in place of the layer obtained by mixing a compound E-2 and a donor compound (Liq: lithium quinolinol). The results are shown in Table 6. E-3 to E-7 are the compounds shown below.

### Examples 63 and 64

In the same manner as in Example 58 except that compounds described in Table 6 were used as an electron transporting layer, light-emitting devices were prepared and evaluated. The results are shown in Table 6. E-8 and E-9 are the compounds shown below.

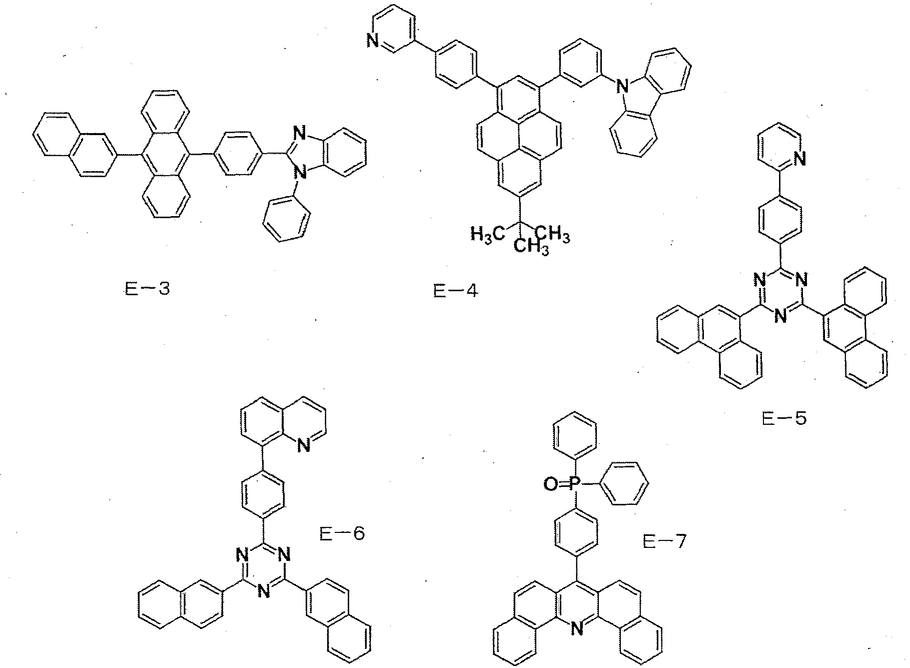

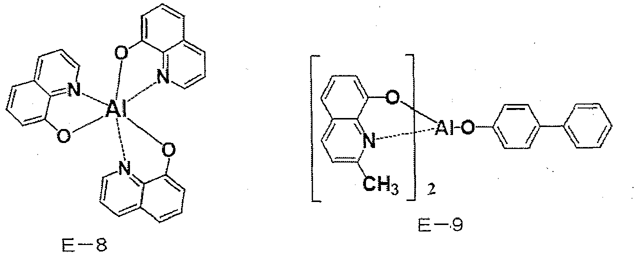

### Example 65

A light-emitting device was prepared in the same manner as in Example 41 except that a compound E-2 and a compound E-1 were laminated in a thickness of 35 nm at a film thickness ratio of 1: 1 and used as an electron transporting layer in place of the layer obtained by mixing a compound E-2 and a donor compound (Liq: lithium quinolinol). The results are shown in Table 6.

### Example 66

A light-emitting device was prepared in the same manner as in Example 41 except that a compound E-3 and a compound E-1 were laminated in a thickness of 35 nm at a film thickness ratio of 1 : 1 and used as an electron transporting layer in place of the layer obtained by mixing a compound E-2 and a donor compound (Liq: lithium quinolinol). The results are shown in Table 6.

### Example 67

A light-emitting device was prepared in the same manner as in Example 41 except that a compound E-4 and a compound E-1 were laminated in a thickness of 35 nm at a film thickness ratio of 1 : 1 and used as an electron transporting layer in place of the layer obtained by mixing a compound E-2 and a donor compound (Liq: lithium quinolinol). The results are shown in Table 6.

**[Table 1]**

| (Examples 3 and 7-10 using compounds 19, 59, 68, 138 and 149 are not forming part of the present invention) | | | | | | |
|---|---|---|---|---|---|---|
| | Second hole transporting layer | Host material | Dopant material | Emitted color | Luminous efficiency (lm/W) | Luminance half-value period (h) |
| Example 1 | Compound [1] | H-1 | D-1 | Blue | 5.1 | 1500 |
| Example 2 | Compound [18] | H-1 | D-1 | Blue | 5.3 | 1600 |
| Example 3 | Compound [19] | H-1 | D-1 | Blue | 4.8 | 1400 |
| Example 4 | Compound [21] | H-1 | D-1 | Blue | 5.3 | 1500 |
| Example 5 | Compound [24] | H-1 | D-1 | Blue | 5.1 | 1500 |
| Example 6 | Compound [4] | H-1 | D-1 | Blue | 5.3 | 1700 |
| Example 7 | Compound [59] | H-1 | D-1 | Blue | 4.5 | 1300 |
| Example 8 | Compound [68] | H-1 | D-1 | Blue | 4.6 | 1300 |
| Example 9 | Compound [138] | H-1 | D-1 | Blue | 4.4 | 1250 |
| Example 10 | Compound [149] | H-1 | D-1 | Blue | 4.5 | 1300 |
| Comparative Example 1 | HT-2 | H-1 | D-1 | Blue | 3.5 | 800 |
| Comparative Example 2 | HT-3 | H-1 | D-1 | Blue | 3.8 | 700 |
| Comparative Example 3 | HT-4 | H-1 | D-1 | Blue | 3.9 | 700 |
| Comparative Example 4 | HT-5 | H-1 | D-1 | Blue | 3.6 | 750 |
| Comparative Example 5 | HT-6 | H-1 | D-1 | Blue | 3.6 | 760 |
| Comparative Example 6 | HT-7 | H-1 | D-1 | Blue | 3.6 | 800 |
| Comparative Example 7 | HT-8 | H-1 | D-1 | Blue | 2.1 | 500 |
| Comparative Example 8 | HT-9 | H-1 | D-1 | Blue | 2.5 | 350 |

**[Table 2]**

| | Second hole transporting layer | Host material | Dopant material | Emitted color | Luminous efficiency (lm/W) | Luminance half-value period (h) |
|---|---|---|---|---|---|---|
| Example 11 | Compound [1] | H-2 | D-2 | Green | 14.0 | 1400 |
| Example 12 | Compound [18] | H-2 | D-2 | Green | 14.5 | 1500 |
| Example 13 | Compound [19] | H-2 | D-2 | Green | 13.5 | 1300 |
| Example 14 | Compound [21] | H-2 | D-2 | Green | 15.0 | 1600 |
| Example 15 | Compound [24] | H-2 | D-2 | Green | 14.0 | 1400 |
| Example 16 | Compound [17] | H-2 | D-2 | Green | 15.0 | 1600 |
| Example 17 | Compound [4] | H-2 | D-2 | Green | 15.0 | 1800 |
| Example 18 | Compound [113] | H-2 | D-2 | Green | 13.0 | 1250 |
| Example 19 | Compound [173] | H-2 | D-2 | Green | 13.0 | 1300 |
| Comparative Example 9 | HT-2 | H-2 | D-2 | Green | 9.6 | 900 |
| Comparative Example 10 | HT-3 | H-2 | D-2 | Green | 8.5 | 600 |
| Comparative Example 11 | HT-4 | H-2 | D-2 | Green | 10.3 | 700 |
| Comparative Example 12 | HT-5 | H-2 | D-2 | Green | 7.5 | 600 |
| Comparative Example 13 | HT-6 | H-2 | D-2 | Green | 9.4 | 900 |
| Comparative Example 14 | HT-8 | H-2 | D-2 | Green | 5.1 | 300 |
| Comparative Example 15 | HT-10 | H-2 | D-2 | Green | 5.5 | 200 |

### (Examples 13, 18 and 19 from Table 2 using compounds 19, 113 and 173 are not forming part of the present invention)

**[Table 3] (Examples 13, 18 and 19 using compounds 19, 113 and 173 are not forming part of the present invention)**

| | Hole transporting layer | Host material | Dopant material | Emitted color | Luminous efficiency (lm/W) | Luminance half-value period (h) |
|---|---|---|---|---|---|---|
| Example 20 | HT-1 | Compound [1] | D-2 | Green | 17.2 | 1400 |
| Example 21 | HT-1 | Compound [18] | D-2 | Green | 18.5 | 1600 |
| Example 22 | HT-1 | Compound [21] | D-2 | Green | 19.0 | 1600 |
| Example 23 | HT-1 | Compound [88] | D-2 | Green | 16.0 | 1350 |
| Example 24 | HT-1 | Compound [170] | D-2 | Green | 16.0 | 1300 |
| Comparative Example 16 | HT-1 | H-3 | D-2 | Green | 8.5 | 550 |
| Comparative Example 17 | HT-1 | H-4 | D-2 | Green | 9.1 | 560 |
| Comparative Example 18 | HT-1 | HT-3 | D-2 | Green | 7.5 | 700 |
| Comparative Example 19 | HT-1 | HT-4 | D-2 | Green | 9.5 | 750 |
| Comparative Example 20 | HT-1 | HT-6 | D-2 | Green | 8.0 | 750 |
| Comparative Example 21 | HT-1 | H-5 | D-2 | Green | 8.5 | 600 |
| Comparative Example 22 | HT-1 | H-6 | D-2 | Green | 9.0 | 700 |
| Comparative Example 23 | HT-1 | HT-8 | D-2 | Green | 9.5 | 800 |
| Comparative Example 24 | HT-1 | HT-10 | D-2 | Green | 9.4 | 830 |

Examples 27 and 32 from Table 4 using compounds 19 and 57 are not forming part of the present invention.

Examples 38 and 39 from Table 5 using compounds 44 and 62 are not forming part of the present invention.

Examples 44, 45, 51, 56 and 57 from Table 6 using compounds 100, 149, 78, 90 and 117 are not forming part of the present invention.

**[Table 4]**

| | Second hole transporting layer | Host material | Dopant material | Emitted color | Luminous efficiency (lm/W) | Luminance half-value period (h) |
|---|---|---|---|---|---|---|
| Example 25 | Compound [1] | H-7 | D-3 | Red | 10.5 | 1400 |
| Example 26 | Compound [18] | H-7 | D-3 | Red | 11.3 | 1500 |
| Example 27 | Compound [19] | H-7 | D-3 | Red | 9.8 | 1300 |
| Example 28 | Compound [21] | H-7 | D-3 | Red | 11.2 | 1500 |
| Example 29 | Compound [24] | H-7 | D-3 | Red | 10.2 | 1400 |
| Example 30 | Compound [4] | H-7 | D-3 | Red | 11.0 | 1500 |
| Example 31 | Compound [7] | H-7 | D-3 | Red | 11.0 | 1500 |
| Example 32 | Compound [57] | H-7 | D-3 | Red | 10.0 | 1200 |
| Comparative Example 25 | HT-2 | H-7 | D-3 | Red | 5.6 | 900 |
| Comparative Example 26 | HT-3 | H-7 | D-3 | Red | 5.5 | 800 |
| Comparative Example 27 | HT-5 | H-7 | D-3 | Red | 5.0 | 900 |
| Comparative Example 28 | HT-7 | H-7 | D-3 | Red | 5.4 | 950 |
| Comparative Example 29 | HT-10 | H-7 | D-3 | Red | 3.5 | 600 |
| Comparative Example 30 | HT-11 | H-7 | D-3 | Red | 3.1 | 500 |

**[Table 5]**

| | Hole injection layer | Second hole transporting layer | Host material | Dopant material | Electron transporting layer | Emitted color | Luminous efficiency (lm/W) | Luminance half-value period (h) |
|---|---|---|---|---|---|---|---|---|
| Example 33 | HI-1 | Compound [1] | H-8 | D-4 | E-2/Liq | Green | 46.0 | 4500 |
| Example 34 | HI-1 | Compound [18] | H-8 | D-4 | E-2/Liq | Green | 50.0 | 5500 |
| Example 35 | HI-1 | Compound [21] | H-8 | D-4 | E-2/Liq | Green | 49.0 | 5500 |
| Example 36 | HI-1 | Compound [4] | H-8 | D-4 | E-2/Liq | Green | 53.0 | 5800 |
| Example 37 | HI-1 | Compound [6] | H-8 | D-4 | E-2/Liq | Green | 50.0 | 5000 |
| Example 38 | HI-1 | Compound [44] | H-8 | D-4 | E-2/Liq | Green | 43.0 | 4000 |
| Example 39 | HI-1 | Compound [62] | H-8 | D-4 | E-2/Liq | Green | 41.0 | 4000 |
| Comparative Example 31 | HI-1 | HT-3 | H-8 | D-4 | E-2/Liq | Green | 28.0 | 2800 |
| Comparative Example 32 | HI-1 | HT-5 | H-8 | D-4 | E-2/Liq | Green | 28.0 | 2700 |
| Comparative Example 33 | HI-1 | HT-6 | H-8 | D-4 | E-2/Liq | Green | 29.0 | 3000 |
| Comparative Example 34 | HI-1 | HT-7 | H-8 | D-4 | E-2/Liq | Green | 23.0 | 2500 |
| Comparative Example 35 | HI-1 | HT-12 | H-8 | D-4 | E-2/Liq | Green | 21.0 | 2500 |
| Comparative Example 36 | HI-1 | HT-8 | H-8 | D-4 | E-2/Liq | Green | 19.0 | 1500 |
| Comparative Example 37 | HI-1 | HT-11 | H-8 | D-4 | E-2/Liq | Green | 18.0 | 1600 |

**[Table 6]**

| | Hole injection layer | Second hole transporting layer | Host material | Dopant material | Electron transporting layer | Emitted color | Luminous efficiency (lm/W) | Luminance half-value period (h) |
|---|---|---|---|---|---|---|---|---|
| Example 40 | HT-12/HI-2 | Compound [1] | H-8 | D-4 | E-2/Liq | Green | 51.0 | 5500 |
| Example 41 | HT-12/HI-2 | Compound [18] | H-8 | D-4 | E-2/Liq | Green | 55.0 | 6100 |
| Example 42 | HT-12/HI-2 | Compound [21] | H-8 | D-4 | E-2/Liq | Green | 55.0 | 6000 |
| Example 43 | HT-12/HI-2 | Compound [4] | H-8 | D-4 | E-2/Liq | Green | 55.0 | 6200 |
| Example 44 | HT-12/HI-2 | Compound [100] | H-8 | D-4 | E-2/Liq | Green | 41.0 | 4000 |
| Example 45 | HT-12/HI-2 | Compound [149] | H-8 | D-4 | E-2/Liq | Green | 48.0 | 4800 |
| Example 46 | HT-12/HI-2 | Compound [1] | H-8/H-9 | D-4 | E-2/Liq | Green | 41.0 | 4100 |
| Example 47 | HT-12/HI-2 | Compound [18] | H-8/H-9 | D-4 | E-2/Liq | Green | 45.0 | 4500 |
| Example 48 | HT-12/HI-2 | Compound [21] | H-8/H-9 | D-4 | E-2/Liq | Green | 45.0 | 4400 |
| Example 49 | HT-12/HI-2 | Compound [4] | H-8/H-9 | D-4 | E-2/Liq | Green | 45.0 | 45P0 |
| Example 50 | HT-12/HI-2 | Compound [3] | H-8/H-9 | D-4 | E-2/Liq | Green | 45.0 | 4600 |
| Example 51 | HT-12/HI-2 | Compound [78] | H-8/H-9 | D-4 | E-2/Liq | Green | 36.0 | 3500 |
| Example 52 | HI-3 | Compound [18] | H-8 | D-4 | E-2/Liq | Green | 38.0 | 4000 |
| Example 53 | HI-3 | Compound [18] | H-8 | D-4 | E-2/Liq | Green | 40.0 | 4300 |
| Example 54 | HI-3 | Compound [21] | H-8 | D-4 | E-2/Liq | Green | 40.0 | 4,200 |
| Example 55 | HI-3 | Compound [4] | H-8 | D-4 | E-2/Liq | Green | 42.0 | 4300 |
| Example 56 | HI-3 | Compound [90] | H-8 | D-4 | E-2/Liq | Green | 32.0 | 3700 |
| Example 57 | HI-3 | Compound [117] | H-8 | D-4 | E-2/Liq | Green | 32.0 | 3700 |
| Example 58 | HT-12/HI-2 | Compound [18] | H-8 | D-4 | E-3 | Green | 51.0 | 5900 |
| Example 59 | HT-12/HI-2 | Compound [18] | H-8 | D-4 | E-4 | Green | 50.0 | 5400 |
| Example 60 | HT-12/HI-2 | Compound [18] | H-8 | D-4 | E-5 | Green | 50.0 | 5800 |
| Example 61 | HT-12/HI-2 | Compound [18] | H-8 | D-4 | E-6/Liq | Green | 51.0 | 6000 |
| Example 62 | HT-12/HI-2 | Compound [18] | H-8 | D-4 | E-7/Liq | Green | 48.0 | 6000 |
| Example 63 | HT-12/HI-2 | Compound [18] | H-8 | D-4 | E-8 | Green | 30.0 | 4000 |
| Example 64 | HT-12/HI-2 | Compound [18] | H-8 | D-4 | E-9 | Green | 27.0 | 3300 |
| Example 65 | HT-12/HI-2 | Compound [18] | H-8 | D-4 | E-2/E-1 | Green | 47.0 | 5700 |
| Example 66 | HT-12/HI-2 | Compound [18] | H-8 | D-4 | E-3/E-1 | Green | 44.0 | 4700 |
| Example 67 | HT-12/HI-2 | Compound [18] | H-8 | D-4 | E-4/E-1 | Green | 46.0 | 5600 |

## Claims

1. A light-emitting device material comprising a compound having a carbazole skeleton and represented by the following general formula (1): 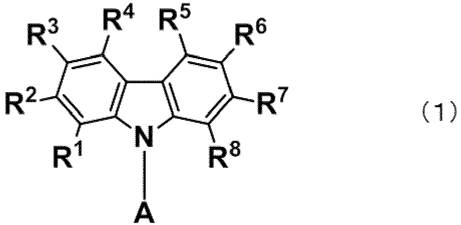 wherein R¹, R² and R⁴ to R⁸ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group, a carbamoyl group, an amino group, a silyl group and -P(=O)R⁹R¹⁰; R⁹ and R¹⁰ are each an aryl group or a heteroaryl group, wherein R³ is a group represented by the general formula (3), and is directly bonded to the position of R¹⁵, R¹, R² and R⁴ to R⁸ contain none of a dibenzofuran skeleton, a dibenzothiophene skeleton and a carbazole skeleton, and R¹, R² and R⁴ to R¹⁰ contain none of an anthracene skeleton and a pyrene skeleton; A is a group represented by any of the following general formulae (6) to (9) and (11) to (16): 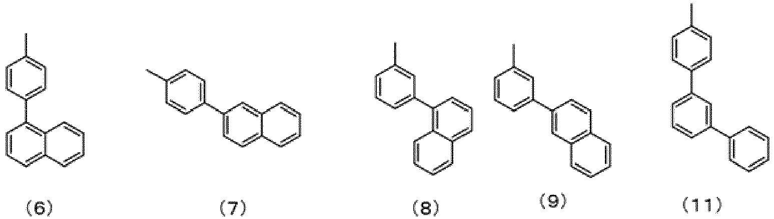 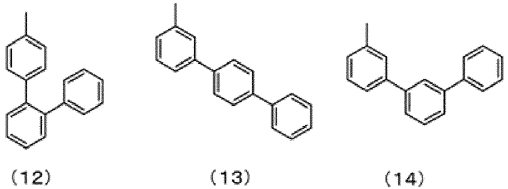 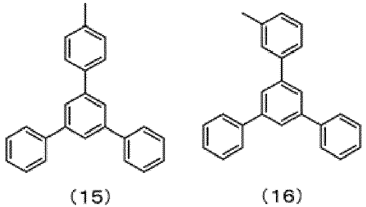 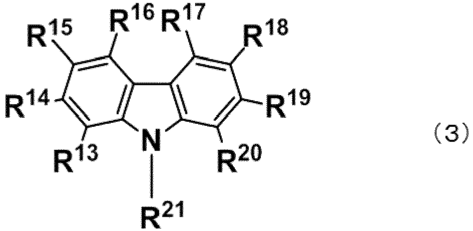
wherein R¹³, R¹⁴ and R¹⁶ to R²⁰ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, halogen, a carbonyl group, a carboxyl group, an oxycarbonyl group, a carbamoyl group, an amino group, a silyl group and - P(=O)R²²R²³; and R²² and R²³ are each an aryl group or a heteroaryl group, wherein R¹⁵ is a group represented by the general formula (1), and is directly bonded to the position of R³;
R¹³, R¹⁴ and R¹⁶ to R²⁰ contain none of a dibenzofuran skeleton, a dibenzothiophene skeleton and a carbazole skeleton, and R¹³, R¹⁴ and R¹⁶ to R²⁰, R²² and R²³ contain none of an anthracene skeleton and a pyrene skeleton, wherein in the general formula (1), A and R²¹ are different groups, wherein R²¹ is a substituted or unsubstituted phenyl group or a substituted or unsubstituted naphthyl group and the substituent is an alkyl group or halogen.

2. A light-emitting device which has an organic layer between an anode and a cathode and emits light by means of electric energy, wherein the light-emitting device contains the light-emitting device material according to claim 1 in any of the layers between the anode and the cathode.

3. A light-emitting device which has at least a hole transporting layer between an anode and a cathode and emits light by means of electric energy, wherein the light-emitting device contains the light-emitting device material according to claim 1 in the hole transporting layer.

4. A light-emitting device which has at least an emissive layer between an anode and a cathode and emits light by means of electric energy, wherein the light-emitting device contains the light-emitting device material according to claim 1 in the emissive layer, and contains a triplet emissive material in the emissive layer.

5. The light-emitting device according to claim 4, wherein the emissive layer has a host material and a triplet emissive dopant material, and the light-emitting device material according to claim 1 is a host material.

6. The light-emitting device according to any one of claims 2 to 5, wherein a hole injection layer exists between the hole transporting layer and the anode, and the hole injection layer contains an acceptor compound.

7. The light-emitting device according to any one of claims 2 to 6, wherein at least an electron transporting layer exists between the emissive layer and the cathode, and the electron transporting layer contains a compound containing electron-accepting nitrogen and having a heteroaryl ring structure composed of elements selected from carbon, hydrogen, nitrogen, oxygen, silicon and phosphorus.

## Patentansprüche

1. Material für lichtemittierende Vorrichtungen, umfassend eine Verbindung mit einem Carbazolgerüst, dargestellt durch die folgende allgemeine Formel (1): 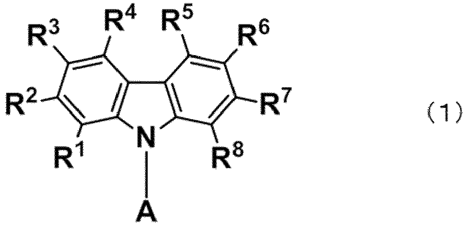 wobei R¹, R² und R⁴ bis R⁸ gleich oder verschieden sein können, und jeweils aus der Gruppe, bestehend aus Wasserstoff, einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einer Arylgruppe, einer Arylgruppe, einer Heteroarylgruppe, Halogen, einer Carbonylgruppe, einer Carboxylgruppe, einer Oxycarbonylgruppe, einer Carbamoylgruppe, einer Aminogruppe, einer Silylgruppe und -P(=O)R⁹R¹⁰ ausgewählt sind, wobei R⁹ und R¹⁰ jeweils eine Arylgruppe oder eine Heteroarylgruppe sind, wobei R³ eine durch die allgemeine Formel (3) dargestellte Gruppe ist und direkt an die Position von R¹⁵ gebunden ist, R¹, R² und R⁴ bis R⁸ kein Dibenzofurangerüst, kein Dibenzothiophengerüst und kein Carbazolgerüst enthalten und R¹, R² und R⁴ bis R¹⁰ kein Anthracengerüst und kein Pyrengerüst enthalten; A eine Gruppe ist, die durch eine der folgenden allgemeinen Formeln (6) bis (9) und (11) bis (16) dargestellt ist: 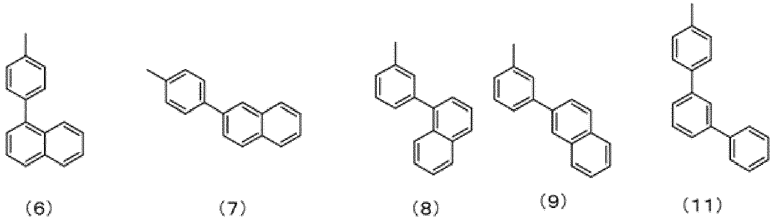 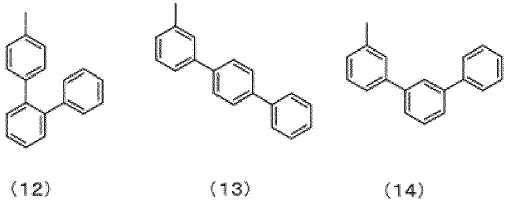 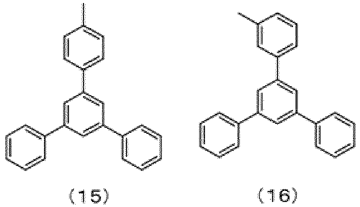 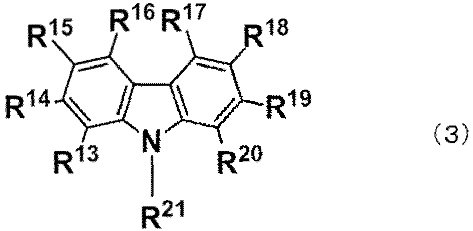 wobei R¹³, R¹⁴ und R⁶ bis R²⁰ gleich oder verschieden sein können, und jeweils aus der Gruppe, bestehend aus Wasserstoff, einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einer Arylgruppe, Halogen, einer Carbonylgruppe, einer Carboxylgruppe, einer Oxycarbonylgruppe, einer Carbamoylgruppe, einer Aminogruppe, einer Silylgruppe und -P (= O) R²² R²³ ausgewählt sind; und R²² und R²³ jeweils eine Arylgruppe oder eine Heteroarylgruppe sind, wobei R¹⁵ eine durch die allgemeine Formel (1) dargestellte Gruppe ist und direkt an die Position von R³ gebunden ist; R¹³, R¹⁴ und R¹⁶ bis R²⁰ kein Dibenzofuran-Gerüst, kein Dibenzothiophen-Gerüst und kein Carbazol-Gerüst enthalten, und R¹³, R¹⁴ und R¹⁶ bis R²⁰, R²² und R²³ kein Anthracen-Gerüst und kein Pyren-Gerüst enthalten, wobei in der allgemeinen Formel (1) A und R²¹ verschiedene Gruppen sind, wobei R²¹ eine substituierte oder unsubstituierte Phenylgruppe oder eine substituierte oder unsubstituierte Naphthylgruppe ist und der Substituent eine Alkylgruppe oder ein Halogen ist.

2. Lichtemittierende Vorrichtung, die eine organische Schicht zwischen einer Anode und einer Kathode aufweist und Licht mittels elektrischer Energie emittiert, wobei die lichtemittierende Vorrichtung das Material für lichtemittierende Vorrichtungen gemäß Anspruch 1 in einer der Schichten zwischen der Anode enthält und die Kathode enthält.

3. Lichtemittierende Vorrichtung, die zumindest eine Öffnungstransportschicht zwischen einer Anode und einer Kathode aufweist und Licht mittels elektrischer Energie emittiert, wobei die lichtemittierende Vorrichtung das Material für lichtemittierende Vorrichtungen nach Anspruch 1 in der Öffnungstransportschicht enthält.

4. Lichtemittierende Vorrichtung, die zumindest eine emittierende Schicht zwischen einer Anode und einer Kathode aufweist und Licht mittels elektrischer Energie emittiert, wobei die lichtemittierende Vorrichtung das Material für lichtemittierende Vorrichtungen gemäß Anspruch 1 in der emittierenden Schicht enthält, und ein Triplett-Emissionsmaterial in der Emissionsschichte enthält.

5. Lichtemittierende Vorrichtung nach Anspruch 4, wobei die emittierende Schicht ein Wirtsmaterial und ein Triplett-emittierendes Dotiermaterial aufweist und das Material für lichtemittierende Vorrichtungen nach Anspruch 1 ein Wirtsmaterial ist.

6. Lichtemittierende Vorrichtung nach einem der Ansprüche 2 bis 5, wobei eine Öffnungsinjektionsschicht zwischen der Öffnungstransportschicht und der Anode vorhanden ist, und die Öffnungsinjektionsschicht eine Akzeptorverbindung enthält.

7. Lichtemittierende Vorrichtung nach einem der Ansprüche 2 bis 6, wobei zumindest eine Elektronentransportschicht zwischen der emittierenden Schicht und der Kathode vorhanden ist und die Elektronentransportschicht eine Verbindung enthält, die elektronenaufnehmenden Stickstoff enthält und die eine Heteroaryl-Ringstruktur bestehend aus Elementen ausgewählt aus Kohlenstoff, Wasserstoff, Stickstoff, Sauerstoff, Silizium und Phosphor aufweist.

## Revendications

1. Matériau pour dispositif électroluminescent comprenant un composé ayant un squelette carbazole et représenté par la formule générale (1) suivante : 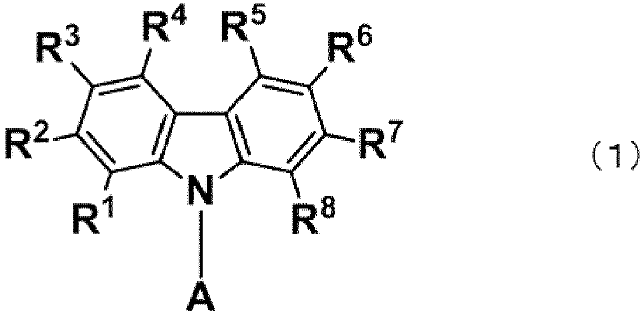 où R¹, R² et R⁴ à R⁸ peuvent être identiques ou différents, et sont chacun choisis dans le groupe constitué par un hydrogène, un groupe alkyle, un groupe cycloalkyle, un groupe hétérocyclique, un groupe alcényle, un groupe cycloalcényle, un groupe alcynyle, un groupe alcoxy, un groupe alkylthio, un groupe aryléther, un groupe arylthioéther, un groupe aryle, un groupe hétéroaryle, un halogène, un groupe carbonyle, un groupe carboxyle, un groupe oxycarbonyle, un groupe carbamoyle, un groupe amino, un groupe silyle et -P(=O)R⁹R¹⁰ ; R⁹ et R¹⁰ représentent chacun un groupe aryle ou un groupe hétéroaryle, où R³ représente un groupe représenté par la formule générale (3), et est directement lié à la position de R¹⁵, R¹, R² et R⁴ à R⁸ ne contiennent aucun squelette parmi un squelette dibenzofurane, un squelette dibenzothiophène et un squelette carbazole, et R¹, R² et R⁴ à R¹⁰ ne contiennent aucun squelette parmi un squelette anthracène et un squelette pyrène ; A représente un groupe représenté par l'une des formules générales (6) à (9) et (11) à (16) suivantes : 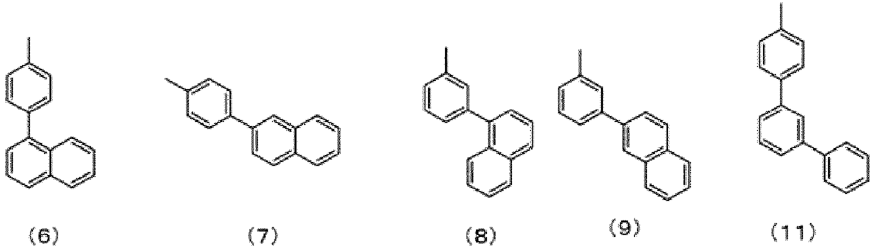 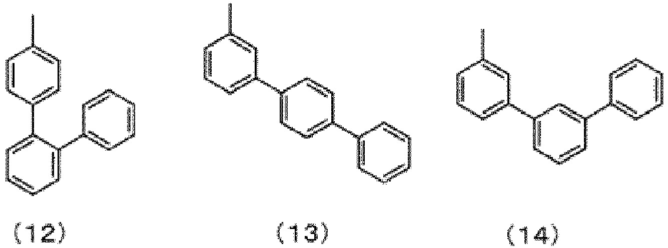 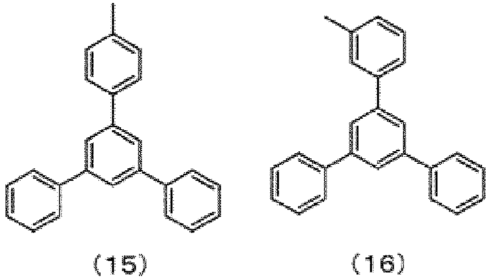 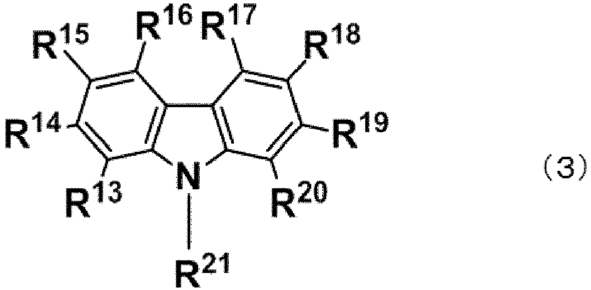
où R¹³, R¹⁴ et R¹⁶ à R²⁰ peuvent être identiques ou différents, et sont chacun choisis dans le groupe constitué par un hydrogène, un groupe alkyle, un groupe cycloalkyle, un groupe hétérocyclique, un groupe alcényle, un groupe cycloalcényle, un groupe alcynyle, un groupe alcoxy, un groupe alkylthio, un groupe aryléther, un groupe arylthioéther, un groupe aryle, un halogène, un groupe carbonyle, un groupe carboxyle, un groupe oxycarbonyle, un groupe carbamoyle, un groupe amino, un groupe silyle et -P(=O)R²²R²³ ; et R²² et R²³ représentent chacun un groupe aryle ou un groupe hétéroaryle, où R¹⁵ représente un groupe représenté par la formule générale (1), et est directement lié à la position de R³;
R¹³, R¹⁴ et R¹⁶ à R²⁰ ne contiennent aucun squelette parmi un squelette dibenzofurane, un squelette dibenzothiophène et un squelette carbazole, et R¹³, R¹⁴ et R¹⁶ à R²⁰, R²² et R²³ ne contiennent aucun squelette parmi un squelette anthracène et un squelette pyrène, où dans la formule générale (1), A et R²¹ sont des groupes différents, où R²¹ représente un groupe phényle substitué ou non substitué ou un groupe naphtyle substitué ou non substitué et le substituant est un groupe alkyle ou un halogène.

2. Dispositif électroluminescent qui a une couche organique entre une anode et une cathode et qui émet de la lumière à l'aide de l'énergie électrique, où le dispositif électroluminescent contient le matériau pour dispositif électroluminescent selon la revendication 1 dans l'une des couches entre l'anode et la cathode.

3. Dispositif électroluminescent qui a au moins une couche de transport de trous entre une anode et une cathode et qui émet de la lumière à l'aide de l'énergie électrique, où le dispositif électroluminescent contient le matériau pour dispositif électroluminescent selon la revendication 1 dans la couche de transport de trous.

4. Dispositif électroluminescent qui a au moins une couche émissive entre une anode et une cathode et qui émet de la lumière à l'aide de l'énergie électrique, où le dispositif électroluminescent contient le matériau pour dispositif électroluminescent selon la revendication 1 dans la couche émissive, et contient un matériau émissif à l'état triplet dans la couche émissive.

5. Dispositif électroluminescent selon la revendication 4, dans lequel la couche émissive a un matériau hôte et un matériau dopant émissif à l'état triplet, et le matériau pour dispositif électroluminescent selon la revendication 1 est un matériau hôte.

6. Dispositif électroluminescent selon l'une quelconque des revendications 2 à 5, dans lequel une couche d'injection de trous existe entre la couche de transport de trous et l'anode, et la couche d'injection de trous contient un composé accepteur.

7. Dispositif électroluminescent selon l'une quelconque des revendications 2 à 6, dans lequel au moins une couche de transport d'électrons existe entre la couche émissive et la cathode, et la couche de transport d'électrons contient un composé contenant de l'azote accepteur d'électrons et ayant une structure de noyau hétéroaryle composée d'éléments choisis parmi le carbone, l'hydrogène, l'azote, l'oxygène, le silicium et le phosphore.
